# EUROPÄISCHE PATENTSCHRIFT

(11) **EP 1 784 649 B1**
(45) Veröffentlichungstag und Bekanntmachung des Hinweises auf die Patenterteilung: **04.05.2011**
(21) Anmeldenummer: 05786960.4
(22) Anmeldetag: 01.09.2005
(51) Int. Cl.: G01N 33/68

(54) **POLYPEPTIDMARKER ZUR DIAGNOSE VON ARTERIOSKLEROSE**
POLYPEPTIDE MARKER FOR THE DIAGNOSIS OF ARTERIOSCLEROSIS
MARQUEURS POLYPEPTIDIQUES POUR LE DIAGNOSTIC DE L'ARTERIOSCLEROSE

(30) Priorität: 02.09.2004 DE 102004042530
(43) Veröffentlichungstag der Anmeldung: 16.05.2007
(73) Patentinhaber: mosaiques diagnostics and therapeutics AG, 30625 Hannover (DE)
(72) Erfinder: MISCHAK, Harald, 31319 Sehnde (DE)
(74) Vertreter: von Kreisler Selting Werner
(86) Internationale Anmeldenummer: PCT/EP2005/054314
(87) Internationale Veröffentlichungsnummer: WO 2006/024660

(56) Entgegenhaltungen:
- US-A1- 2003 170 745
- KAISER T ET AL: "Capillary electrophoresis coupled to mass spectrometer for automated and robust pofypaptide determination in body fluids for clinical use" ELECTROPHORESIS, WEINHEIM, DE, Bd. 25, Nr. 13, Juli 2004 (2004-07), Seiten 2044-2055, XP002304343 ISSN: 0173-0835
- WEISSINGER E M ET AL: "Proteomic patterns established with capillary electrophoresis and mass spectrometry for diagnostic purposes" KIDNEY INTERNATIONAL, NEW YORK, NY, US, Bd. 65, Nr. 6, Juni 2004 (2004-06), Seiten 2426-2434, XP002304344 ISSN: 0085-2538
- YOU S-A ET AL: "Proteomic approach to coronary atherosclerosis shows ferritin light chain as a significant marker: Evidence consistent with iron hypothesis in atherosclerosis" PHYSIOLOGICAL GENOMICS, AMERICAN PHYSIOLOGICAL SOCIETY, BETHESDA, MD, US, Bd. 13, Juli 2003 (2003-07), Seiten 25-30, XP002315211 ISSN: 1094-8341

## Beschreibung

Die vorliegende Erfindung betrifft die Verwendung der An- oder Abwesenheit eines oder mehrerer Peptidmarker in einer Probe eines Individuums zur Diagnose von Arteriosklerose sowie ein Verfahren zur Diagnose von Arteriosklerose, wobei die An- oder Abwesenheit des oder der Peptidmarker(s) indikativ für das Vorliegen von Arteriosklerose ist.

Arteriosklerose - oder auch Atherosklerose, Gefäß- bzw. Arterienverkalkung - ist die häufigste krankhafte Gefäßveränderung. Arteriosklerose ist eine Veränderung der Blutgefäße, die über viele Jahre entsteht und zunächst unerkannt verläuft. An den Gefäßwänden reichern sich Blutfette und weiße Blutkörperchen an (Plaquebildung), die Gefäße verkalken, verlieren ihre Elastizität und der Gefäßdurchmesser verengt sich zunehmend. Als Risikofaktoren für die Ausbildung einer Arteriosklerose gelten Hypercholesterinämie, Bluthochdruck, Rauchen, Diabetes mellitus, Fettsucht, körperliche Inaktivität und ein niedriger sozialer Status, der jedoch eng mit anderen Faktoren verknüpft ist. Weitere Risikofaktoren wie Hypertriglyceridämie, erhöhte Blutkonzentrationen von Lipoprotein (a), Homocystein und bestimmten Entzündungsparametern wie c-reaktives Protein (CRP) oder Fibrinogen sowie chronische Chlamydien- oder Helicobacter pylori-Infektion werden diskutiert.

Die Arteriosklerose verursacht lange keine Symptome. Erst wenn der Gefäßdurchmesser durch die Plaques deutlich reduziert wurde oder sich im Bereich des Plaques ein Blutgerinnsel (Thrombus) bildet, kommt es zu Symptomen. Die häufigsten arteriosklerotischen Herz- und Gefäßerkrankungen sind die koronare Herzkrankheit, zerebrovaskuläre Erkrankungen und periphere arterielle Verschlusskrankheit, deren gefürchtete Komplikationen, also ein Myokardinfarkt, ein Schlaganfall oder der Verlust der unteren Extremitäten, kommen in Deutschland im Vergleich zu ärmeren Ländern außerordentlich häufig vor und verursachen extrem hohe Kosten für das Gesundheitswesen.

Arteriosklerose-bedingte Herz-Kreislauf-Erkrankungen, auch als kardiovaskuläre Erkrankungen bezeichnet, stehen in der deutschen Todesursachenstatistik an vorderster Stelle. So bildeten kardiovaskuläre Erkrankungen 1999 nach Angabe des Statistischen Bundesamts mit 48% die häufigste Todesursache, wobei ischämische (koronare) Herzkrankheiten 21% und Krankheiten des zerebrovaskulären Systems 10% ausmachten. Im Vergleich dazu betrug der Anteil von Krebserkrankungen mit Todesfolge 26%, bei Krankheiten des Atmungs- und Verdauungssystems betrug der Anteil 6% bzw. 5%.

Obwohl arteriosklerotische Veränderungen oft bereits im Jugendalter stattfinden, werden die Patienten meist erst ab einem mittleren und höheren Lebensalter klinisch auffällig.

Die Arteriosklerose kann nicht durch Medikamente behandelt, sondern nur durch Vorbeugung vermieden werden. Bereits eingetreten Verkalkungen können nicht abgebaut werden, den starren Gefäßwänden kann die Elastizität nicht zurückgegeben werden. Allerdings kann das Fortschreiten der Arteriosklerose deutlich verlangsamt werden, indem die Risikofaktoren durch Änderung der Lebensweise, etwa durch Nikotinabstinenz, oder medikamentös (z.B. durch Acetylsalicylsäure) beeinflusst werden. Daneben stehen bei schweren Arterienverkalkungen auch chirurgische Verfahren, wie z.B. Ballonerweiterung (PTA, perkutane transluminale Angioplastie), Bypass-Operation und das Einsetzen von Stents zur Verfügung.

Da, wie bereits erläutert, arteriosklerotische Veränderung nicht therapeutisch behandelt, sondern nur vermieden oder verlangsamt werden können, ist eine frühzeitige Erkennung arteriosklerotischer Veränderungen besonders wichtig.

Nach Sun-Ah You et al. (Physiol. Genomics, Bd. 13:25-30, 2003) kann Ferritin als Marker für Arteriosklerose genutzt werden.

US 2003/170745 A1 beschreibt CAP37 als Marker für chronisch entzündliche Prozesse wie z.B. Arteriosklerose.

Kaiser et al. beschreiben ein Verfahren zur Auswertung von Urin- und Serumproben bei Diabetes, Sepsis und chronischen Nierenkrankheiten (Electrophoresis, Bd.25:2044-2055, 2004).

Weissinger et al. beschreiben, dass Urin- und Serumproben nach diesem Verfahren zur Diagnostik der Erkrankung minimal change disease (MCD), der Fokal-segmentalen Glomerulosklerose (FSGS) und der Membranösen Glomerulonephritis (MGN) herangezogen werden können (Kidney International, Bd. 65:2426-2434, 2004).

Unter normalen Umständen lässt erst die Diagnose einer Folgeerkrankung die Schlussfolgerung zu, dass der Betroffene an Arteriosklerose erkrankt ist. Gegenwärtig werden hierzu die Verengungen der Blutgefäße z.B. mit Hilfe von Kontrastmitteln oder Röntgen oder mittels Ultraschall nachgewiesen. Darüber hinaus werden für gewöhnlich die Risikofaktoren, wie z.B. erhöhte Cholesterinwerte oder eine Zuckerkrankheit, untersucht. Alle diese Verfahren sind aber nicht ausreichend für eine frühzeitige und zuverlässige Diagnose der Arteriosklerose. Insbesondere besteht ein Bedarf an einer möglichst wenig invasiven, schnellen und kostengünstigen Früherkennung der Arteriosklerose.

Überraschender Weise wurde nun gefunden, dass bestimmte Peptidmarker in der Probe eines Individuums zur Diagnose von Arteriosklerose verwendet werden können.

Folglich ist ein Gegenstand der vorliegenden Erfindung die Verwendung der An- oder Abwesenheit mindestens eines Polypeptidmarkers in einer Probe eines Individuums zur

Diagnose von Arteriosklerose, wobei der Polypeptidmarker ausgewählt ist aus den Polypeptidmarkem Nr. 1, Nr. 2, Nr. 3, Nr. 4, Nr. 5, Nr. 6, Nr. 7, Nr. 8 oder Nr. 9, die durch die in Tabelle 2 angegebenen Werte für die Molekularmasse und für die Migrationszeiten charakterisiert sind:

**Tabelle 2: Polypeptidmarker zur Diagnose von Arteriosklerose sowie ihre Molekularmassen und Migrationszeiten**

| Polypeptidmarker Nr. | Molekularmasse [Da] | Migrationszeit [min] |
|---|---|---|
| 1 | 2511,8 | 26,4 |
| 2 | 1864,7 | 49,2 |
| 3 | 2799,8 | 46,0 |
| 4 | 1340,6 | 66,7 |
| 5 | 1422,8 | 61,2 |
| 6 | 5882,0 | 33,3 |
| 7 | 1397,4 | 24,9 |
| 8 | 1886,6 | 44,7 |
| 9 | 4642,6 | 37,5 |

Mit der vorliegenden Erfindung ist es möglich, Arteriosklerose sehr frühzeitig zu diagnostizieren. Dadurch kann die Krankheit in einem frühen Stadium oder vor ihrer Manifestierung durch bekannte therapeutische Ansätze behandelt werden und so in ihrem weiteren Verlauf gemildert werden. Die Erfindung ermöglicht weiterhin eine kostengünstige, schnelle und zuverlässige Diagnose der Arteriosklerose bei zum Teil nicht oder nur minimal invasivem Eingriffen.

Die Migrationszeit wird mittels Kapillarelektrophorese (capillary electrophoresis, CE) - z.B. wie in Beispiel unter Punkt 2 ausgeführt - bestimmt. Hierbei wird eine 90 cm lange Glaskapillare mit einem inneren Durchmesser (ID) von 75 µm und einem äußeren Durchmesser (OD) von 360 µm bei einer Spannung von 30 kV betrieben. Als Lösungsmittel für die Probe wird 30% Methanol, 0,5% Ameisensäure in Wasser verwendet.

Es ist bekannt, dass die CE-Migrationszeit variieren kann. Dennoch ist die Reihenfolge, mit der die Polypeptidmarker eluieren, für jedes verwendete CE-System typischerweise gleich. Um die Unterschiede in der Migrationszeit auszugleichen, kann das System unter Verwendung von Standards, für die die Migrationszeiten bekannt sind, normiert werden. Diese Standards können z.B. die in den Beispielen angegebenen Polypeptide sein (siehe Beispiel Punkt 3 "Standards für die CE-Messung").

Die Charakterisierung der Polypeptidmarker, die in den Tabellen 2 und 3 gezeigt sind, wurde mittels Kapillarelektrophorese-Massenspektrometrie (capillary electrophoresis-mass spectrometry, CE-MS) bestimmt, einem Verfahren, das zum Beispiel ausführlich von Neuhoff et al. (Rapid Communications in mass spectrometry, Bd. 20:149-156, 2004) beschrieben wurde. Die Variation der Molekularmassen zwischen einzelnen Messungen oder zwischen verschiedenen Massenspektrometern ist relativ klein, typischerweise ist sie im Bereich von ± 0,1%, vorzugsweise im Bereich von ± 0,05%.

Die erfindungsgemäßen Polypeptidmarker sind Proteine oder Peptide oder Abbauprodukte von Proteinen oder Peptiden. Sie können chemisch modifiziert sein, z.B. durch posttranslationale Modifikationen, wie Glycosylierung, Phosphorylierung, Alkylierung oder Disulfidbrücken, oder durch andere Reaktionen, z.B. im Rahmen des Abbaus, verändert sein. Darüber hinaus können die Polypeptidmarker auch im Rahmen der Aufreinigung der Proben chemisch verändert, z.B. oxidiert, sein.

Ausgehend von den Parametern, die die Polypeptidmarker (Molekularmasse und Migrationszeit) bestimmen, ist es möglich, durch im Stand der Technik bekannte Verfahren die Sequenz der entsprechenden Polypeptide zu identifizieren.

Die erfindungsgemäßen Polypeptide (siehe Tabelle 2) werden verwendet, um Arteriosklerose zu diagnostizieren. Unter Diagnose versteht man den Vorgang der Erkenntnisgewinnung durch die Zuordnung von Symptomen oder Phänomenen zu einer Krankheit oder Verletzung. Im vorliegenden Fall wird von der An- oder Abwesenheit bestimmter Polypeptidmarker auf das Vorliegen von Arteriosklerose geschlossen. Hierzu werden die erfindungsgemäßen Polypeptidmarker in einer Probe eines Individuums bestimmt, wobei ihre Anoder Abwesenheit auf das Vorliegen von Arteriosklerose schließen lässt. Die An- oder Abwesenheit eines Polypeptidmarkers kann durch jedes im Stand der Technik bekannte Verfahren gemessen werden. Verfahren, die verwendet werden könne, sind weiter unter beispielhaft aufgeführt.

Ein Polypeptidmarker ist anwesend, wenn sein Messwert mindestens so hoch ist wie der des Schwellenwerts. Liegt der Messwert darunter, ist der Polypeptidmarker abwesend. Der Schwellenwert kann entweder durch die Sensitivität des Messverfahrens (Nachweisgrenze) bestimmt werden oder anhand von Erfahrungen definiert werden.

Im Zusammenhang mit der vorliegenden Erfindung wird der Schwellenwert vorzugsweise überschritten, wenn der Messwert der Probe für eine bestimmte Molekularmasse mindestens doppelt so hoch ist wie der einer Leerprobe (z.B. nur Puffer oder Lösungsmittel), weiter bevorzugt ist der Messwert mindestens dreimal so hoch, noch weiter bevorzugt mindestens viermal so hoch und am meisten bevorzugt mindestens fünfmal so hoch wie der der Leerprobe.

Der oder die Polypeptidmarker wird/werden in der Weise verwendet, dass seine/ihre An- oder Abwesenheit gemessen wird, wobei die An- oder Abwesenheit indikativ für Arteriosklerose ist. So gibt es Polypeptidmarker, die typischerweise bei Patienten mit Arteriosklerose (krank) vorhanden sind, wie z.B. Polypeptidmarker Nr. 1, Nr. 7, Nr. 8 und Nr. 9, jedoch bei Probanden ohne Arteriosklerose (Kontrolle) nicht vorhanden sind. Darüber hinaus gibt es Polypeptidmarker, die bei Individuen ohne Arteriosklerose (Kontrolle) vorhanden sind, jedoch bei Individuen mit Arteriosklerose seltener oder gar nicht auftreten. Dies sind z.B. die Polypeptidmarker Nr. 2, Nr. 3, Nr. 4, Nr. 5 und Nr. 6.

**Tabelle 3: Polypeptidmarker zur Diagnose von Arteriosklerose, ihre Molekularmassen und Migrationszeiten sowie ihre An- und Abwesenheit bei an Arteriosklerose erkrankten Patienten und Kontrollen (Probenaufbereitung und Messung wie im Beispiel beschrieben):**

| Polypeptidmarker Nr. | Molekularmasse [Da] | Migrationszeit [min] | krank | Kontrolle | Δ |
|---|---|---|---|---|---|
| 1 | 2511,8 | 26,4 | 0,60 | 0,00 | 0,60 |
| 2 | 1864,7 | 49,2 | 0,20 | 0,78 | 0,58 |
| 3 | 2799,8 | 46,0 | 0,20 | 0,78 | 0,58 |
| 4 | 1340,6 | 66,7 | 0,10 | 0,67 | 0,57 |
| 5 | 1422,8 | 61,2 | 0,00 | 0,56 | 0,56 |
| 6 | 5882,0 | 33,3 | 0,00 | 0,56 | 0,56 |
| 7 | 1397,4 | 24,9 | 0,50 | 0,00 | 0,50 |
| 8 | 1886,6 | 44,7 | 0,50 | 0,00 | 0,50 |
| 9 | 4642,6 | 37,5 | 0,50 | 0,00 | 0,50 |

| | | | | | |
|---|---|---|---|---|---|
| krank Anteil der Individuen mit Arteriosklerose, bei denen der Polypeptidmarker in der Probe vorhanden war Kontrolle Anteil der nicht an Arteriosklerose erkrankten Individuen, bei denen der Polypeptidmarker in der Probe vorhanden war Δ Betrag von (krank - Kontrolle) | | | | | |

Das Individuum, von dem die Probe stammt, in der die An- oder Abwesenheit eines oder mehrerer Polypeptidmarker bestimmt wird, kann jedes Individuum sein, das an Arteriosklerose leiden kann, z.B. ein Tier oder ein Mensch. Vorzugsweise handelt es sich bei dem Individuum um ein Säugetier, wie z.B. einen Hund, ein Pferd, eine Katze, und am meisten bevorzugt handelt es sich um einen Menschen.

In einer bevorzugten Ausführungsform der Erfindung wird nicht nur ein Polypeptidmarker, sondern eine Kombination von Polypeptidmarkern verwendet, um Arteriosklerose zu diagnostizieren, wobei durch ihre An- oder Abwesenheit auf das Vorliegen von Arteriosklerose rückgeschlossen werden kann. Durch Vergleich einer Mehrzahl von Polypeptidmarkern kann die Verfälschung des Gesamtergebnisses durch einzelne individuelle Abweichungen von der typischen Anwesenheitswahrscheinlichkeit im Kranken oder Kontrollindividuum reduziert oder vermieden werden.

In einer bevorzugten Ausführungsform der Erfindung werden daher zwei Polypeptidmarker der Tabelle 2 zur Diagnose von Arteriosklerose verwendet. Insbesondere sind dies die Kombinationen der Polypeptidmarker:
- Nr. 1 und Nr. 2, Nr. 1 und Nr. 3, Nr. 1 und Nr. 4, Nr. 1 und Nr. 5, Nr. 1 und Nr. 6, Nr. 1 und Nr. 7, Nr. 1 und Nr. 8, Nr. 1 und Nr. 9,
- Nr. 2 und Nr. 3, Nr. 2 und Nr. 4, Nr. 2 und Nr. 5, Nr. 2 und Nr. 6, Nr. 2 und Nr. 7, Nr. 2 und Nr. 8, Nr. 2 und Nr. 9,
- Nr. 3 und Nr. 4, Nr. 3 und Nr. 5, Nr. 3 und Nr. 6, Nr. 3 und Nr. 7, Nr. 3 und Nr. 8, Nr. 3 und Nr. 9,
- Nr. 4 und Nr. 5, Nr. 4 und Nr. 6, Nr. 4 und Nr. 7, Nr. 4 und Nr. 8, Nr. 4 und Nr. 9,
- Nr. 5 und Nr. 6, Nr. 5 und Nr. 7, Nr. 5 und Nr. 8, Nr. 5 und Nr. 9,
- Nr. 6 und Nr. 7, Nr. 6 und Nr. 8, Nr. 6 und Nr. 9,
- Nr. 7 und Nr. 8, Nr. 7 und Nr. 9 oder
- Nr. 8 und Nr. 9.

In einer anderen, noch weiter bevorzugten Ausführungsform der Erfindung werden drei Polypeptidmarker der Tabelle 2 zur Diagnose von Arteriosklerose verwendet. Insbesondere sind dies die Kombinationen der Polypeptidmarker:
- Nr. 1 und Nr. 2 in Kombination mit einem der Polypeptidmarker Nr. 3, Nr. 4, Nr. 5, Nr. 6, Nr.7, Nr. 8 oder Nr. 9,
- Nr. 1 und Nr. 3 in Kombination mit einem der Polypeptidmarker Nr. 4, Nr. 5, Nr. 6, Nr.7, Nr. 8 oder Nr. 9,
- Nr. 1 und Nr. 4 in Kombination mit einem der Polypeptidmarker Nr. 5, Nr. 6, Nr.7, Nr. 8 oder Nr. 9,
- Nr. 1 und Nr. 5 in Kombination mit einem der Polypeptidmarker Nr. 6, Nr.7, Nr. 8 oder Nr. 9,
- Nr. 1 und Nr. 6 in Kombination mit einem der Polypeptidmarker Nr.7, Nr. 8 oder Nr. 9,
- Nr. 1 und Nr. 7 in Kombination mit einem der Polypeptidmarker Nr. 8 oder Nr. 9,
- Nr. 1 und Nr. 8 in Kombination mit dem Polypeptidmarker Nr. 9,
- Nr. 2 und Nr. 3 in Kombination mit einem der Polypeptidmarker Nr. 4, Nr. 5, Nr. 6, Nr.7, Nr. 8 oder Nr. 9,
- Nr. 2 und Nr. 4 in Kombination mit einem der Polypeptidmarker Nr. 5, Nr. 6, Nr.7, Nr. 8 oder Nr. 9,
- Nr. 2 und Nr. 5 in Kombination mit einem der Polypeptidmarker Nr. 6, Nr.7, Nr. 8 oder Nr. 9,
- Nr. 2 und Nr. 6 in Kombination mit einem der Polypeptidmarker Nr.7, Nr. 8 oder Nr. 9,
- Nr. 2 und Nr. 7 in Kombination mit einem der Polypeptidmarker Nr. 8 oder Nr. 9,
- Nr. 2 und Nr. 8 in Kombination mit Nr. 9,
- Nr. 3 und Nr. 4 in Kombination mit einem der Polypeptidmarker Nr. 5, Nr. 6, Nr.7,Nr. 8 oder Nr. 9,
- Nr. 3 und Nr. 5 in Kombination mit einem der Polypeptidmarker Nr. 6, Nr.7, Nr. 8 oder Nr. 9,
- Nr. 3 und Nr. 6 in Kombination mit einem der Polypeptidmarker Nr.7, Nr. 8 oder Nr. 9,
- Nr. 3 und Nr. 7 in Kombination mit einem der Polypeptidmarker Nr. 8 oder N r. 9,
- Nr. 3 und Nr. 8 in Kombination mit Nr. 9,
- Nr. 4 und Nr. 5 in Kombination mit einem der Polypeptidmarker Nr. 6, Nr.7, Nr. 8 oder Nr. 9,
- Nr. 4 und Nr. 6 in Kombination mit einem der Polypeptidmarker Nr.7, Nr. 8 oder Nr. 9,
- Nr. 4 und Nr. 7 in Kombination mit einem der Polypeptidmarker Nr. 8 oder N r. 9,
- Nr. 4 und Nr. 8 in Kombination mit Nr. 9,
- Nr. 5 und Nr. 6 in Kombination mit einem der Polypeptidmarker Nr.7, Nr. 8 oder Nr. 9,
- Nr. 5 und Nr. 7 in Kombination mit einem der Polypeptidmarker Nr. 8 oder Nr. 9,
- Nr. 5 und Nr. 8 in Kombination mit Nr. 9,
- Nr. 6 und Nr. 7 in Kombination mit Nr. 8 oder Nr. 9,
- Nr. 6 und Nr. 8 in Kombination mit Nr. 9 oder
- Nr. 7 und Nr. 8 in Kombination mit Nr. 9.

In einer andren noch weiter bevorzugten Ausführungsform der Erfindung werden vier Polypeptidmarker der Tabelle 2 zur Diagnose von Arteriosklerose verwendet. Insbesondere sind dies die Kombinationen der Polypeptidmarker:
- Nr. 1, Nr. 2 und Nr. 3 in Kombination mit einem der Polypeptidmarker Nr. 4, Nr. 5, Nr. 6, Nr.7, Nr. 8 oder Nr. 9,
- Nr. 1, Nr. 2 und Nr. 4 in Kombination mit einem der Polypeptidmarker Nr. 5, Nr. 6, Nr.7, Nr. 8 oder Nr. 9,
- Nr. 1, Nr. 2 und Nr. 5 in Kombination mit einem der Polypeptidmarker Nr. 6, Nr.7, Nr. 8 oder Nr. 9,
- Nr. 1, Nr. 2 und Nr. 6 in Kombination mit einem der Polypeptidmarker Nr.7, Nr. 8 oder Nr. 9,
- Nr. 1, Nr. 2 und Nr. 7 in Kombination mit einem der Polypeptidmarker Nr. 8 oder Nr. 9,
- Nr. 1, Nr. 2 und Nr. 8 in Kombination mit Nr. 9,
- Nr. 1, Nr. 3 und Nr. 4 in Kombination mit einem der Polypeptidmarker Nr. 5, Nr. 6, Nr.7, Nr. 8 oder Nr. 9,
- Nr. 1, Nr. 3 und Nr. 5 in Kombination mit einem der Polypeptidmarker Nr. 6, Nr.7, Nr. 8 oder Nr. 9,
- Nr. 1, Nr. 3 und Nr. 6 in Kombination mit einem der Polypeptidmarker Nr.7, Nr. 8 oder Nr. 9,
- Nr. 1, Nr. 3 und Nr. 7 in Kombination mit einem der Polypeptidmarker Nr. 8 oder Nr. 9,
- Nr. 1, Nr. 3 und Nr. 8 in Kombination mit Nr. 9,
- Nr. 1, Nr. 4 und Nr. 5 in Kombination mit einem der Polypeptidmarker Nr. 6, Nr.7, Nr. 8 oder Nr. 9,
- Nr. 1, Nr. 4 und Nr. 6 in Kombination mit einem der Polypeptidmarker Nr.7, Nr. 8 oder Nr. 9,
- Nr. 1, Nr. 4 und Nr. 7 in Kombination mit einem der Polypeptidmarker Nr. 8 oder Nr. 9,
- Nr. 1, Nr. 4 und Nr. 8 in Kombination mit Nr. 9,
- Nr. 1, Nr. 5 und Nr. 6 in Kombination mit einem der Polypeptidmarker Nr.7, Nr. 8 oder Nr. 9,
- Nr. 1, Nr. 5 und Nr. 7 in Kombination mit einem der Polypeptidmarker Nr. 8 oder Nr. 9,
- Nr. 1, Nr. 5 und Nr. 8 in Kombination mit Nr. 9,
- Nr. 1, Nr. 6 und Nr. 7 in Kombination mit einem der Polypeptidmarker Nr. 8 oder Nr. 9,
- Nr. 1, Nr. 6 und Nr. 8 in Kombination mit Nr. 9,
- Nr. 1, Nr. 7 und Nr. 8 in Kombination mit Nr. 9,
- Nr. 2, Nr. 3 und Nr. 4 in Kombination mit einem der Polypeptidmarker Nr. 5, Nr. 6, Nr.7, Nr. 8 oder Nr. 9,
- Nr. 2, Nr. 3 und Nr. 5 in Kombination mit einem der Polypeptidmarker Nr. 6, Nr.7, Nr. 8 oder Nr. 9,
- Nr. 2, Nr. 3 und Nr. 6 in Kombination mit einem der Polypeptidmarker Nr.7, Nr. 8 oder Nr. 9,
- Nr. 2, Nr. 3 und Nr. 7 in Kombination mit einem der Polypeptidmarker Nr. 8 oder Nr. 9,
- Nr. 2, Nr. 3 und Nr. 8 in Kombination mit Nr. 9,
- Nr. 2, Nr. 4 und Nr. 5 in Kombination mit einem der Polypeptidmarker Nr. 6, Nr.7, Nr. 8 oder Nr. 9,
- Nr. 2, Nr. 4 und Nr. 6 in Kombination mit einem der Polypeptidmarker Nr.7, Nr. 8 oder Nr. 9,
- Nr. 2, Nr. 4 und Nr. 7 in Kombination mit einem der Polypeptidmarker Nr. 8 oder Nr. 9,
- Nr. 2, Nr. 4 und Nr. 8 in Kombination mit Nr. 9,
- Nr. 2, Nr. 5 und Nr. 6 in Kombination mit einem der Polypeptidmarker Nr.7, Nr. 8 oder Nr. 9,
- Nr. 2, Nr. 5 und Nr. 7 in Kombination mit einem der Polypeptidmarker Nr. 8 oder Nr. 9,
- Nr. 2, Nr. 5 und Nr. 8 in Kombination mit Nr. 9,
- Nr. 2, Nr. 6 und Nr. 7 in Kombination mit einem der Polypeptidmarker Nr. 8 oder Nr. 9,
- Nr. 2, Nr. 6 und Nr. 8 in Kombination mit Nr. 9,
- Nr. 2, Nr. 7 und Nr. 8 in Kombination mit Nr. 9,
- Nr. 3, Nr. 4 und Nr. 5 in Kombination mit einem der Polypeptidmarker Nr. 6, Nr.7, Nr. 8 oder Nr. 9,
- Nr. 3, Nr. 4 und Nr. 6 in Kombination mit einem der Polypeptidmarker Nr.7, Nr. 8 oder Nr. 9,
- Nr. 3, Nr. 4 und Nr. 7 in Kombination mit einem der Polypeptidmarker Nr. 8 oder Nr. 9,
- Nr. 3, Nr. 4 und Nr. 8 in Kombination mit Nr. 9,
- Nr. 3, Nr. 5 und Nr. 6 in Kombination mit einem der Polypeptidmarker Nr.7, Nr. 8 oder Nr. 9,
- Nr. 3, Nr. 5 und Nr. 7 in Kombination mit einem der Polypeptidmarker Nr. 8 oder Nr. 9,
- Nr. 3, Nr. 5 und Nr. 8 in Kombination mit Nr. 9,
- Nr. 3, Nr. 6 und Nr. 7 in Kombination mit einem der Polypeptidmarker Nr. 8 oder Nr. 9,
- Nr. 3, Nr. 6 und Nr. 8 in Kombination mit Nr. 9,
- Nr. 3, Nr. 7 und Nr. 8 in Kombination mit Nr. 9,
- Nr. 4, Nr. 5 und Nr. 6 in Kombination mit einem der Polypeptidmarker Nr.7, Nr. 8 oder Nr. 9,
- Nr. 4, Nr. 5 und Nr. 7 in Kombination mit einem der Polypeptidmarker Nr. 8 oder Nr. 9,
- Nr. 4, Nr. 5 und Nr. 8 in Kombination mit Nr. 9,
- Nr. 4, Nr. 6 und Nr. 7 in Kombination mit einem der Polypeptidmarker Nr. 8 oder Nr. 9,
- Nr. 4, Nr. 6 und Nr. 8 in Kombination mit Nr. 9,
- Nr. 4, Nr. 7 und Nr. 8 in Kombination mit Nr. 9,
- Nr. 5, Nr. 6 und Nr. 7 in Kombination mit einem der Polypeptidmarker Nr. 8 oder Nr. 9,
- Nr. 5, Nr. 6 und Nr. 8 in Kombination mit Nr. 9,
- Nr. 5, Nr. 7 und Nr. 8 in Kombination mit Nr. 9 oder
- Nr. 6, Nr. 7 und Nr. 8 in Kombination mit Nr. 9.

In einer noch weiter bevorzugten Ausführungsform der Erfindung werden fünf Polypeptidmarker der Tabelle 2 zur Diagnose von Arteriosklerose verwendet. Insbesondere sind dies die Kombinationen der Polypeptidmarker:
- Nr. 1, Nr. 2, Nr. 3 und Nr. 4 in Kombination mit einem der Polypeptidmarker Nr. 5, Nr. 6, Nr.7, Nr. 8 oder Nr. 9,
- Nr. 1, Nr. 2, Nr. 3 und Nr. 5 in Kombination mit einem der Polypeptidmarker Nr. 6, Nr.7, Nr. 8 oder Nr. 9,
- Nr. 1, Nr. 2, Nr. 3 und Nr. 6 in Kombination mit einem der Polypeptidmarker Nr.7, Nr. 8 oder Nr. 9,
- Nr. 1, Nr. 2, Nr. 3 und Nr. 7 in Kombination mit einem der Polypeptidmarker Nr. 8 oder Nr. 9,
- Nr. 1, Nr. 2, Nr. 3 und Nr. 8 in Kombination mit Polypeptidmarker Nr. 9,
- Nr. 1, Nr. 2, Nr. 4 und Nr. 5 in Kombination mit einem der Polypeptidmarker Nr. 6, Nr.7, Nr. 8 oder Nr. 9,
- Nr. 1, Nr. 2, Nr. 4 und Nr. 6 in Kombination mit einem der Polypeptidmarker Nr.7, Nr. 8 oder Nr. 9,
- Nr. 1, Nr. 2, Nr. 4 und Nr. 7 in Kombination mit einem der Polypeptidmarker Nr. 8 oder Nr. 9,
- Nr. 1, Nr. 2, Nr. 4 und Nr. 8 in Kombination mit Polypeptidmarker Nr. 9,
- Nr. 1, Nr. 2, Nr. 5 und Nr. 6 in Kombination mit einem der Polypeptidmarker Nr.7, Nr. 8 oder Nr. 9,
- Nr. 1, Nr. 2, Nr. 5 und Nr. 7 in Kombination mit einem der Polypeptidmarker Nr. 8 oder Nr. 9,
- Nr. 1, Nr. 2 und Nr. 5, Nr. 8 in Kombination mit Polypeptidmarker Nr. 9,
- Nr. 1, Nr. 2 und Nr. 6, Nr. 7 in Kombination mit einem der Polypeptidmarker Nr. 8 oder Nr. 9,
- Nr. 1, Nr. 2, Nr. 6 und Nr. 8 in Kombination mit Polypeptidmarker Nr. 9,
- Nr. 1, Nr. 2, Nr. 7 und Nr. 8 in Kombination mit Polypeptidmarker Nr. 9,
- Nr. 1, Nr. 3, Nr. 4 und Nr. 5 in Kombination mit einem der Polypeptidmarker Nr. 6, Nr.7, Nr. 8 oder Nr. 9,
- Nr. 1, Nr. 3, Nr. 4 und Nr. 6 in Kombination mit einem der Polypeptidmarker Nr.7, Nr. 8 oder Nr. 9,
- Nr. 1, Nr. 3, Nr. 4 und Nr. 7 in Kombination mit einem der Polypeptidmarker Nr. 8 oder Nr. 9,
- Nr. 1, Nr. 3, Nr. 4 und Nr. 8 in Kombination mit Polypeptidmarker Nr. 9,
- Nr. 1, Nr. 3, Nr. 5 und Nr. 6 in Kombination mit einem der Polypeptidmarker Nr.7, Nr. 8 oder Nr. 9,
- Nr. 1, Nr. 3, Nr. 5 und Nr. 7 in Kombination mit einem der Polypeptidmarker Nr. 8 oder Nr. 9,
- Nr. 1, Nr. 3, Nr. 5 und Nr. 8 in Kombination mit Polypeptidmarker Nr. 9,
- Nr. 1, Nr. 3, Nr. 6 und Nr. 7 in Kombination mit einem der Polypeptidmarker Nr. 8 oder Nr. 9,
- Nr. 1, Nr. 3, Nr. 6 und Nr. 8 in Kombination mit Polypeptidmarker Nr. 9,
- Nr. 1, Nr. 3, Nr. 7 und Nr. 8 in Kombination mit Polypeptidmarker Nr. 9,
- Nr. 1, Nr. 4, Nr. 5 und Nr. 6 in Kombination mit einem der Polypeptidmarker Nr.7, Nr. 8 oder Nr. 9,
- Nr. 1, Nr. 4, Nr. 5 und Nr. 7 in Kombination mit einem der Polypeptidmarker Nr. 8 oder Nr. 9,
- Nr. 1, Nr. 4, Nr. 5 und Nr. 8 in Kombination mit Polypeptidmarker Nr. 9,
- Nr. 1, Nr. 4, Nr. 6 und Nr. 7 in Kombination mit einem der Polypeptidmarker Nr. 8 oder Nr. 9,
- Nr. 1, Nr. 4, Nr. 6 und Nr. 8 in Kombination mit Polypeptidmarker Nr. 9,
- Nr. 1, Nr. 4, Nr. 7 und Nr. 8 in Kombination mit Polypeptidmarker Nr. 9,
- Nr. 1, Nr. 5, Nr. 6 und Nr. 7 in Kombination mit einem der Polypeptidmarker Nr. 8 oder Nr. 9,
- Nr. 1, Nr. 5, Nr. 6 und Nr. 8 in Kombination mit Polypeptidmarker Nr. 9,
- Nr. 1, Nr. 5, Nr. 7 und Nr. 8 in Kombination mit Polypeptidmarker Nr. 9,
- Nr. 1, Nr. 6, Nr. 7 und Nr. 8 in Kombination mit Polypeptidmarker Nr. 9,
- Nr. 2, Nr. 3, Nr. 4 und Nr. 5 in Kombination mit einem der Polypeptidmarker Nr. 6, Nr.7, Nr. 8 oder Nr. 9,
- Nr. 2, Nr. 3, Nr. 4 und Nr. 6 in Kombination mit einem der Polypeptidmarker Nr.7, Nr. 8 oder Nr. 9,
- Nr. 2, Nr. 3, Nr. 4 und Nr. 7 in Kombination mit einem der Polypeptidmarker Nr. 8 oder Nr. 9,
- Nr. 2, Nr. 3, Nr. 4 und Nr. 8 in Kombination mit Polypeptidmarker Nr. 9,
- Nr. 2, Nr. 3, Nr. 5 und Nr. 6 in Kombination mit einem der Polypeptidmarker Nr.7, Nr. 8 oder Nr. 9,
- Nr. 2, Nr. 3, Nr. 5 und Nr. 7 in Kombination mit einem der Polypeptidmarker Nr. 8 oder Nr. 9,
- Nr. 2, Nr. 3, Nr. 5 und Nr. 8 in Kombination mit Polypeptidmarker Nr. 9,
- Nr. 2, Nr. 3, Nr. 6 und Nr. 7 in Kombination mit einem der Polypeptidmarker Nr. 8 oder Nr. 9,
- Nr. 2, Nr. 3, Nr. 6 und Nr. 8 in Kombination mit Polypeptidmarker Nr. 9,
- Nr. 2, Nr. 3, Nr. 7 und Nr. 8 in Kombination mit Polypeptidmarker Nr. 9,
- Nr. 2, Nr. 4, Nr. 5 und Nr. 6 in Kombination mit einem der Polypeptidmarker Nr.7, Nr. 8 oder Nr. 9,
- Nr. 2, Nr. 4, Nr. 5 und Nr. 7 in Kombination mit einem der Polypeptidmarker Nr. 8 oder Nr. 9,
- Nr. 2, Nr. 4, Nr. 5 und Nr. 8 in Kombination mit Polypeptidmarker Nr. 9,
- Nr. 2, Nr. 4, Nr. 6 und Nr. 7 in Kombination mit einem der Polypeptidmarker Nr. 8 oder Nr. 9,
- Nr. 2, Nr. 4, Nr. 6 und Nr. 8 in Kombination mit Polypeptidmarker Nr. 9,
- Nr. 2, Nr. 4, Nr. 7 und Nr. 8 in Kombination mit Polypeptidmarker Nr. 9,
- Nr. 2, Nr. 5, Nr. 6 und Nr. 7 in Kombination mit einem der Polypeptidmarker Nr. 8 oder Nr. 9,
- Nr. 2, Nr. 5, Nr. 6 und Nr. 8 in Kombination mit Polypeptidmarker Nr. 9,
- Nr. 2, Nr. 5, Nr. 7 und Nr. 8 in Kombination mit Polypeptidmarker Nr. 9,
- Nr. 2, Nr. 6, Nr. 7 und Nr. 8 in Kombination mit Polypeptidmarker Nr. 9,
- Nr. 3, Nr. 4, Nr. 5 und Nr. 6 in Kombination mit einem der Polypeptidmarker Nr.7, Nr. 8 oder Nr. 9,
- Nr. 3, Nr. 4, Nr. 5 und Nr. 7 in Kombination mit einem der Polypeptidmarker Nr. 8 oder Nr. 9,
- Nr. 3, Nr. 4, Nr. 5 und Nr. 8 in Kombination mit Polypeptidmarker Nr. 9,
- Nr. 3, Nr. 4, Nr. 6 und Nr. 7 in Kombination mit einem der Polypeptidmarker Nr. 8 oder Nr. 9,
- Nr. 3, Nr. 4, Nr. 6 und Nr. 8 in Kombination mit Polypeptidmarker Nr. 9,
- Nr. 3, Nr. 4, Nr. 7 und Nr. 8 in Kombination mit Polypeptidmarker Nr. 9,
- Nr. 3, Nr. 5, Nr. 6 und Nr. 7 in Kombination mit einem der Polypeptidmarker Nr. 8 oder Nr. 9,
- Nr. 3, Nr. 5, Nr. 6 und Nr. 8 in Kombination mit Polypeptidmarker Nr. 9,
- Nr. 3, Nr. 5, Nr. 7 und Nr. 8 in Kombination mit Polypeptidmarker Nr. 9,
- Nr. 3, Nr. 6, Nr. 7 und Nr. 8 in Kombination mit Polypeptidmarker Nr. 9,
- Nr. 4, Nr. 5, Nr. 6 und Nr. 7 in Kombination mit einem der Polypeptidmarker Nr. 8 oder Nr. 9,
- Nr. 4, Nr. 5, Nr. 6 und Nr. 8 in Kombination mit Polypeptidmarker Nr. 9,
- Nr. 4, Nr. 5, Nr. 7 und Nr. 8 in Kombination mit Polypeptidmarker Nr. 9,
- Nr. 4, Nr. 6, Nr. 7 und Nr. 8 in Kombination mit Polypeptidmarker Nr. 9 oder
- Nr. 5, Nr. 6, Nr. 7 und Nr. 8 in Kombination mit Polypeptidmarker Nr. 9.

In einer noch weiter bevorzugten Ausführungsform der Erfindung werden sechs Polypeptidmarker der Tabelle 2 zur Diagnose von Arteriosklerose verwendet. Insbesondere sind dies die Kombinationen der Polypeptidmarker:
- Nr. 1, Nr. 2, Nr. 3, Nr. 4 und Nr. 5 in Kombination mit einem der Polypeptidmarker Nr. 6, Nr.7, Nr. 8 oder Nr. 9,
- Nr. 1, Nr. 2, Nr. 3, Nr. 4 und Nr. 6 in Kombination mit einem der Polypeptidmarker Nr. 7, Nr. 8 oder Nr. 9,
- Nr. 1, Nr. 2, Nr. 3, Nr. 4 und Nr. 7 in Kombination mit einem der Polypeptidmarker Nr. 8 oder Nr. 9,
- Nr. 1, Nr. 2, Nr. 3, Nr. 4 und Nr. 8 in Kombination mit Polypeptidmarker N r. 9,
- Nr. 1, Nr. 2, Nr. 3, Nr. 5 und Nr. 6 in Kombination mit einem der Polypeptidmarker Nr. 7, Nr. 8 oder Nr. 9,
- Nr. 1, Nr. 2, Nr. 3, Nr. 5 und Nr. 7 in Kombination mit einem der Polypeptidmarker Nr. 8 oder Nr. 9,
- Nr. 1, Nr. 2, Nr. 3, Nr. 5 und Nr. 8 in Kombination mit Polypeptidmarker Nr. 9,
- Nr. 1, Nr. 2, Nr. 3, Nr. 6 und Nr. 7 in Kombination mit einem der Polypeptidmarker Nr. 8 oder Nr. 9,
- Nr. 1, Nr. 2, Nr. 3, Nr. 6 und Nr. 8 in Kombination mit Polypeptidmarker Nr. 9,
- Nr. 1, Nr. 2, Nr. 3, Nr. 7 und Nr. 8 in Kombination mit Polypeptidmarker Nr. 9,
- Nr. 1, Nr. 2, Nr. 4, Nr. 5 und Nr. 6 in Kombination mit einem der Polypeptidmarker Nr.7, Nr. 8 oder Nr. 9,
- Nr. 1, Nr. 2, Nr. 4, Nr. 5 und Nr. 7 in Kombination mit einem der tidmarker Nr. 8 oder Nr. 9,
- Nr. 1, Nr. 2, Nr. 4, Nr. 5 und Nr. 8 in Kombination mit Polypeptidmarker N r. 9,
- Nr. 1, Nr. 2, Nr. 4, Nr. 6 und Nr. 7 in Kombination mit einem der Polypeptidmarker Nr. 8 oder Nr. 9,
- Nr. 1, Nr. 2, Nr. 4, Nr. 6 und Nr. 8 in Kombination mit Polypeptidmarker Nr. 9,
- Nr. 1, Nr. 2, Nr. 4, Nr. 7 und Nr. 8 in Kombination mit Polypeptidmarker N r. 9,
- Nr. 1, Nr. 2, Nr. 5, Nr. 6 und Nr. 7 in Kombination mit einem der Polypeptidmarker Nr. 8 oder Nr. 9,
- Nr. 1, Nr. 2, Nr. 5, Nr. 6 und Nr. 8 in Kombination mit Polypeptidmarker Nr. 9,
- Nr. 1, Nr. 2, Nr. 5, Nr. 7 und Nr. 8 in Kombination mit Polypeptidmarker Nr. 9,
- Nr. 1, Nr. 2, Nr. 6, Nr. 7 und Nr. 8 in Kombination mit Polypeptidmarker Nr. 9,
- Nr. 1, Nr. 3, Nr. 4, Nr. 5 und Nr. 6 in Kombination mit einem der Polypeptidmarker Nr.7, Nr. 8 oder Nr. 9,
- Nr. 1, Nr. 3, Nr. 4, Nr. 5 und Nr. 7 in Kombination mit einem der Polypeptidmarker Nr. 8 oder Nr. 9,
- Nr. 1, Nr. 3, Nr. 4, Nr. 5 und Nr. 8 in Kombination mit Polypeptidmarker N r. 9,
- Nr. 1, Nr. 3, Nr. 4, Nr. 6 und Nr. 7 in Kombination mit einem der Polypeptidmarker Nr. 8 oder Nr. 9,
- Nr. 1, Nr. 3, Nr. 4, Nr. 6 und Nr. 8 in Kombination mit Polypeptidmarker Nr. 9,
- Nr. 1, Nr. 3, Nr. 4, Nr. 7 und Nr. 8 in Kombination mit Polypeptidmarker - 20 - N r. 9,
- Nr. 1, Nr. 3, Nr. 5, Nr. 6 und Nr. 7 in Kombination mit einem der Polypeptidmarker Nr. 8 oder Nr. 9,
- Nr. 1, Nr. 3, Nr. 5, Nr. 6 und Nr. 8 in Kombination mit Polypeptidmarker Nr. 9,
- Nr. 1, Nr. 3, Nr. 5, Nr. 7 und Nr. 8 in Kombination mit Polypeptidmarker Nr. 9,
- Nr. 1, Nr. 3, Nr. 6, Nr. 7 und Nr. 8 in Kombination mit Polypeptidmarker Nr. 9,
- Nr. 1, Nr. 4, Nr. 5, Nr. 6 und Nr. 7 in Kombination mit einem der Polypeptidmarker Nr. 8 oder Nr. 9,
- Nr. 1, Nr. 4, Nr. 5, Nr. 6 und Nr. 8 in Kombination mit Polypeptidmarker Nr. 9,
- Nr. 1, Nr. 4, Nr. 5, Nr. 7 und Nr. 8 in Kombination mit Polypeptidmarker Nr. 9,
- Nr. 1, Nr. 4, Nr. 6, Nr. 7 und Nr. 8 in Kombination mit Polypeptidmarker Nr. 9,
- Nr. 1, Nr. 5, Nr. 6, Nr. 7 und Nr. 8 in Kombination mit Polypeptidmarker N r. 9,
- Nr. 2, Nr. 3, Nr. 4, Nr. 5 und Nr. 6 in Kombination mit einem der Polypeptidmarker Nr.7, Nr. 8 oder Nr. 9,
- Nr. 2, Nr. 3, Nr. 4, Nr. 5 und Nr. 7 in Kombination mit einem der Polypeptidmarker Nr. 8 oder Nr. 9,
- Nr. 2, Nr. 3, Nr. 4, Nr. 5 und Nr. 8 in Kombination mit Polypeptidmarker Nr. 9,
- Nr. 2, Nr. 3, Nr. 4, Nr. 6 und Nr. 7 in Kombination mit einem der Polypeptidmarker Nr. 8 oder Nr. 9,
- Nr. 2, Nr. 3, Nr. 4, Nr. 6 und Nr. 8 in Kombination mit Polypeptidmarker Nr. 9,
- Nr. 2, Nr. 3, Nr. 4, Nr. 7 und Nr. 8 in Kombination mit Polypeptidmarker Nr. 9,
- Nr. 2, Nr. 3, Nr. 5, Nr. 6 und Nr. 7 in Kombination mit einem der Polypeptidmarker Nr. 8 oder Nr. 9,
- Nr. 2, Nr. 3, Nr. 5, Nr. 6 und Nr. 8 in Kombination mit Polypeptidmarker Nr. 9,
- Nr. 2, Nr. 3, Nr. 5, Nr. 7 und Nr. 8 in Kombination mit Polypeptidmarker Nr. 9,
- Nr. 2, Nr. 3, Nr. 6, Nr. 7 und Nr. 8 in Kombination mit Polypeptidmarker Nr. 9,
- Nr. 2, Nr. 4, Nr. 5, Nr. 6 und Nr. 7 in Kombination mit einem der Polypeptidmarker Nr. 8 oder Nr. 9,
- Nr. 2, Nr. 4, Nr. 5, Nr. 6 und Nr. 8 in Kombination mit Polypeptidmarker N r. 9,
- Nr. 2, Nr. 4, Nr. 5, Nr. 7 und Nr. 8 in Kombination mit Polypeptidmarker Nr. 9,
- Nr. 2, Nr. 4, Nr. 6, Nr. 7 und Nr. 8 in Kombination mit Polypeptidmarker Nr. 9,
- Nr. 2, Nr. 5, Nr. 6, Nr. 7 und Nr. 8 in Kombination mit Polypeptidmarker Nr. 9,
- Nr. 3, Nr. 4, Nr. 5, Nr. 6 und Nr. 7 in Kombination mit einem der Polypeptidmarker Nr. 8 oder Nr. 9,
- Nr. 3, Nr. 4, Nr. 5, Nr. 6 und Nr. 8 in Kombination mit Polypeptidmarker Nr. 9,
- Nr. 3, Nr. 4, Nr. 5, Nr. 7 und Nr. 8 in Kombination mit Polypeptidmarker Nr. 9,
- Nr. 3, Nr. 4, Nr. 6, Nr. 7 und Nr. 8 in Kombination mit Polypeptidmarker Nr. 9,
- Nr. 3, Nr. 5, Nr. 6, Nr. 7 und Nr. 8 in Kombination mit Polypeptidmarker Nr. 9 oder
- Nr. 4, Nr. 5, Nr. 6, Nr. 7 und Nr. 8 in Kombination mit Polypeptidmarker Nr. 9.

In einer noch weiter bevorzugten Ausführungsform der Erfindung werden sieben Polypeptidmarker der Tabelle 2 zur Diagnose von Arteriosklerose verwendet. Insbesondere sind dies die Kombinationen der Polypeptidmarker:
- Nr. 1, Nr. 2, Nr. 3, Nr. 4, Nr. 5 und Nr. 6 in Kombination mit einem der Polypeptidmarker Nr. 7, Nr. 8 oder Nr. 9,
- Nr. 1, Nr. 2, Nr. 3, Nr. 4, Nr. 5 und Nr. 7 in Kombination mit einem der Polypeptidmarker Nr. 8 oder Nr. 9,
- Nr. 1, Nr. 2, Nr. 3, Nr. 4, Nr. 5 und Nr. 8 in Kombination mit Polypeptidmarker Nr. 9,
- Nr. 1, Nr. 2, Nr. 3, Nr. 4, Nr. 6 und Nr. 7 in Kombination mit einem der Polypeptidmarker Nr. 8 oder Nr. 9,
- Nr. 1, Nr. 2, Nr. 3, Nr. 4, Nr. 6 und Nr. 8 in Kombination mit Polypeptidmarker Nr. 9,
- Nr. 1, Nr. 2, Nr. 3, Nr. 4, Nr. 7 und Nr. 8 in Kombination mit Polypeptidmarker Nr. 9,
- Nr. 1, Nr. 2, Nr. 3, Nr. 5, Nr. 6 und Nr. 7 in Kombination mit einem der Polypeptidmarker Nr. 8 oder Nr. 9,
- Nr. 1, Nr. 2, Nr. 3, Nr. 5, Nr. 6 und Nr. 8 in Kombination mit Polypeptidmarker Nr. 9,
- Nr. 1, Nr. 2, Nr. 3, Nr. 5, Nr. 7 und Nr. 8 in Kombination mit Polypeptidmarker Nr. 9,
- Nr. 1, Nr. 2, Nr. 3, Nr. 6, Nr. 7 und Nr. 8 in Kombination mit Polypeptidmarker Nr. 9,
- Nr. 1, Nr. 2, Nr. 4, Nr. 5, Nr. 6 und Nr. 7 in Kombination mit einem der Polypeptidmarker Nr. 8 oder Nr. 9,
- Nr. 1, Nr. 2, Nr. 4, Nr. 5, Nr. 6 und Nr. 8 in Kombination mit Polypeptidmarker Nr. 9,
- Nr. 1, Nr. 2, Nr. 4, Nr. 5, Nr. 7 und Nr. 8 in Kombination mit Polypeptidmarker Nr. 9,
- Nr. 1, Nr. 2, Nr. 4, Nr. 6, Nr. 7 und Nr. 8 in Kombination mit Polypeptidmarker Nr. 9,
- Nr. 1, Nr. 2, Nr. 5, Nr. 6, Nr. 7 und Nr. 8 in Kombination mit Polypeptidmarker Nr. 9,
- Nr. 1, Nr. 3, Nr. 4, Nr. 5, Nr. 6 und Nr. 7 in Kombination mit einem der Polypeptidmarker Nr. 8 oder Nr. 9,
- Nr. 1, Nr. 3, Nr. 4, Nr. 5, Nr. 6 und Nr. 8 in Kombination mit Polypeptidmarker Nr. 9,
- Nr. 1, Nr. 3, Nr. 4, Nr. 5, Nr. 7 und Nr. 8 in Kombination mit Polypeptidmarker Nr. 9,
- Nr. 1, Nr. 3, Nr. 4, Nr. 6, Nr. 7 und Nr. 8 in Kombination mit Polypeptidmarker Nr. 9,
- Nr. 1, Nr. 3, Nr. 5, Nr. 6, Nr. 7 und Nr. 8 in Kombination mit Polypeptidmarker Nr. 9,
- Nr. 1, Nr. 4, Nr. 5, Nr. 6, Nr. 7 und Nr. 8 in Kombination mit Polypeptidmarker Nr. 9,
- Nr. 2, Nr. 3, Nr. 4, Nr. 5, Nr. 6 und Nr. 7 in Kombination mit einem der Polypeptidmarker Nr. 8 oder Nr. 9,
- Nr. 2, Nr. 3, Nr. 4, Nr. 5, Nr. 6 und Nr. 8 in Kombination mit Polypeptidmarker Nr. 9,
- Nr. 2, Nr. 3, Nr. 4, Nr. 5, Nr. 7 und Nr. 8 in Kombination mit Polypeptidmarker Nr. 9,
- Nr. 2, Nr. 3, Nr. 4, Nr. 6, Nr. 7 und Nr. 8 in Kombination mit Polypeptidmarker Nr. 9,
- Nr. 2, Nr. 3, Nr. 5, Nr. 6, Nr. 7 und Nr. 8 in Kombination mit Polypeptidmarker Nr. 9,
- Nr. 2, Nr. 4, Nr. 5, Nr. 6, Nr. 7 und Nr. 8 in Kombination mit Polypeptidmarker Nr. 9 oder
- Nr. 3, Nr. 4, Nr. 5, Nr. 6, Nr. 7 und Nr. 8 in Kombination mit Polypeptidmarker Nr. 9.

In einer noch weiter bevorzugten Ausführungsform der Erfindung werden acht Polypeptidmarker der Tabelle 2 zur Diagnose von Arteriosklerose verwendet. Insbesondere sind dies die Kombinationen der Polypeptidmarker:
- Nr. 1, Nr. 2, Nr. 3, Nr. 4, Nr. 5, Nr. 6, Nr. 7 und Nr. 8,
- Nr. 1, Nr. 2, Nr. 3, Nr. 4, Nr. 5, Nr. 6, Nr. 7 und Nr. 9,
- Nr. 1, Nr. 2, Nr. 3, Nr. 4, Nr. 5, Nr. 6, Nr. 8 und Nr. 9,
- Nr. 1, Nr. 2, Nr. 3, Nr. 4, Nr. 5, Nr. 7, Nr. 8 und Nr. 9,
- Nr. 1, Nr. 2, Nr. 3, Nr. 4, Nr. 6, Nr. 7, Nr. 8 und Nr. 9,
- Nr. 1, Nr. 2, Nr. 3, Nr. 5, Nr. 6, Nr. 7, Nr. 8 und Nr. 9,
- Nr. 1, Nr. 2, Nr. 4, Nr. 5, Nr. 6, Nr. 7, Nr. 8 und Nr. 9,
- Nr. 1, Nr. 3, Nr. 4, Nr. 5, Nr. 6, Nr. 7, Nr. 8 und Nr. 9 oder
- Nr. 2, Nr. 3, Nr. 4, Nr. 5, Nr. 6, Nr. 7, Nr. 8 und Nr. 9.

In einer noch weiter bevorzugten Ausführungsform der Erfindung werden neun Polypeptidmarker der Tabelle 1 oder 2 zur Diagnose von Arteriosklerose verwendet. Insbesondere sind dies die Kombinationen der Polypeptidmarker:
- Nr. 1, Nr. 2, Nr. 3, Nr. 4, Nr. 5, Nr. 6, Nr. 7, Nr. 8 und Nr. 9.

Zusätzlich zu den oben erwähnten Polypeptidmarkern kann die An- oder Abwesenheit mindestens eines weiteren Polypeptids als Marker zur Diagnose von Arteriosklerose verwendet werden, wenn dieses Polypeptid indikativ für Arteriosklerose oder das Nichtvorliegen von Arteriosklerose (Kontrolle, z.B. gesunder Zustand) ist. Das genannte Polypeptid besteht beispielsweise aus mindestens zehn Aminosäuren, die über Peptidbindungen verbunden sind. Vorzugsweise besteht das Polypeptid aus maximal 100 Aminosäuren. Weiter bevorzugt hat das Polypeptid eine Molekularmasse von circa 500 - 15.000 Da, noch weiter bevorzugt von circa 750 - circa. 10.000 Da, insbesondere circa 1.000 - circa 7.500 Da. Hierbei können die Marker chemisch modifiziert sein, z.B. durch posttranslationale Modifikationen, wie Glycosylierung, Phosphorylierung, Alkylierung oder Disulfidbrücken, oder durch andere Reaktionen, z.B. im Rahmen des Abbaus, verändert sein. Darüber hinaus können die Polypeptidmarker auch im Rahmen der Aufreinigung der Proben chemisch verändert, z.B. oxidiert, sein.

Bei der Probe, in der die An- oder Abwesenheit des oder der erfindungsgemäßen Polypeptidmarker gemessen werden, kann es sich um jede Probe handeln, die aus dem Körper des Individuums gewonnen wird. Bei der Probe handelt es sich um eine Probe, die über eine Polypeptidzusammensetzung verfügt, die geeignet ist, Aussagen über den Zustand des Individuums (Arteriosklerose oder gesund, d.h. Individuum ohne Arteriosklerose) zu treffen. Beispielsweise kann es sich um Blut, Urin, eine Gelenkflüssigkeit, eine Gewebeflüssigkeit, ein Körpersekret, Schweiß, Liquor, Lymphe, Darm-, Magen-, Pankreassaft, Galle, Tränenflüssigkeit, eine Gewebeprobe, Sperma, Vaginalflüssigkeit oder eine Stuhlprobe handeln. Vorzugsweise handelt es sich um eine Flüssigprobe.

In einer bevorzugten Ausführungsform handelt es sich bei der Probe um eine Urinprobe oder eine Blutprobe, am meisten bevorzugt ist eine (Blut)serum-oder (Blut)plasmaprobe.

Urinproben können wie im Stand der Technik bevorzugt genommen werden. Vorzugsweise wird im Zusammenhang mit der vorliegenden Erfindung eine Mittelstrahlurinprobe als Urinprobe verwendet. Die Urinprobe kann zum Beispiel auch mittels eines Urinierungsapparats, wie in WO 01/74275 beschrieben, entnommen werden.

Blutproben können durch im Stand der Technik bekannte Verfahren beispielsweise aus einer Vene, Arterie oder Kapillare entnommen werden. Für gewöhnlich wird eine Blutprobe erhalten, indem dem Individuum venöses Blut mittels einer Spritze z.B. aus dem Arm entnommen wird. Der Begriff Blutprobe bezieht auch Proben ein, die aus Blut durch weitere Aufreinigungs- und Trennungsverfahren gewonnen wurden, wie z.B. (Blut)plasma oder (Blut)serum.

Blutplasma wird aus Blut gewonnen, indem die zellulären Bestandteile z.B. durch Zentrifugation entfernt werden. Die Zentrifugation kann beispielsweise in Gegenwart von Gerinnungshemmern, wie z.B. Natriumcitrat, erfolgen, da im Plasma noch die Gerinnungsfaktoren noch vorhanden sind. Blutserum entspricht im Wesentlichen Blutplasma, aus dem die gerinnungsaktiven Proteine - hauptsächlich Fibrinogen - z.B. durch Blutgerinnung entfernt wurden.

Verfahren zur Herstellung von Blutplasma und Blutserum sind im Stand der Technik allgemein bekannt. Darüber hinaus offenbart WO 04/65958 ein Verfahren zum Entfernen von Fibrinogen aus Plasma. Zusammensetzungen zur Trennung von Serum oder Plasma sind z.B. in WO 03/48764 (siehe auch US 2004/129631) offenbart. Vorrichtungen und Verfahren zum Sammeln von Plasma- oder Serumproben aus Blut sind beispielsweise in US 2004/31746 beschrieben.

Die An- oder Abwesenheit eines Polypeptidmarkers in der Probe kann durch jedes im Stand der Technik bekannte Verfahren, das zur Messung von Polypeptidmarkern geeignet ist, bestimmt werden. Dem Fachmann sind solche Verfahren bekannt. Grundsätzlich kann die An- oder Abwesenheit eines Polypeptidmarkers durch direkte Verfahren, wie z.B. Massenspektrometrie, oder indirekte Verfahren, wie z.B. mittels Liganden, bestimmt werden.

Falls erforderlich oder wünschenswert kann die Probe des Individuums, z.B. die Urin- oder Blutprobe, vor der Messung der An- oder Abwesenheit des oder der Polypeptidmarkers durch jedes geeignete Mittel vorbehandelt und z.B. aufgereinigt oder aufgetrennt werden. Die Behandlung kann z.B. eine Aufreinigung, Trennung, Verdünnung oder Konzentrierung umfassen. Die Verfahren können beispielsweise eine Zentrifugation, eine Filtration, eine
Ultrafiltration, eine Dialyse, eine Fällung oder chromatographische Verfahren wie Affinitätstrennung oder Trennung mittels Ionenaustauscherchromatographie, eine elektrophoretische Trennung, d.h. Trennung durch unterschiedliches Wanderungsverhalten elektrisch geladener Teilchen in Lösung beim Anlegen eines elektrischen Feldes, sein. Besondere Beispiele hiervon sind Gelelektrophorese, zweidimensionale Polyacrylamidgel-Elektrophorese (2D-PAGE), Kapillarelektrophorese, Metallaffinitätschromatographie, immobilisierte Metallaffmitätschromatographie (IMAC), Affinitätschromatographie auf Basis von Lektinen, Flüssigchromatographie, Hochleistungsflüssigchromatographie (HPLC), Normal- und Umkehrphasen-HPLC, Kationenaustauscherchromatographie und selektive Bindung an Oberflächen. Alle diese Verfahren sind dem Fachmann gut bekannt und der Fachmann wird das Verfahren in Abhängigkeit von der verwendeten Probe und dem Verfahren zur Bestimmung der An- oder Abwesenheit des oder der Polypeptidmarker auswählen können.

In einer Ausführungsform der Erfindung wird die Probe vor ihrer Trennung mittels Kapillarelektrophorese aufgetrennt, mittels Ultrazentrifugation gereinigt und/oder mittels Ultrafiltration in Fraktionen, die Polypeptidmarker bestimmter molekularer Größe enthalten, aufgeteilt.

Vorzugsweise wird ein massenspektrometrisches Verfahren verwendet, um die An- oder Abwesenheit eines Polypeptidmarkers zu bestimmen, wobei diesem Verfahren eine Aufreinigung oder Auftrennung der Probe vorgeschaltet sein kann. Die massenspektrometrische Analyse besitzt gegenüber den derzeit gängigen Verfahren den Vorteil, dass die Konzentration vieler (>100) Polypeptide einer Probe mittels einer einzigen Analyse bestimmt werden kann. Jeder Typ eines Massenspektrometers kann verwendet werden. Mit der Massenspektrometrie ist es möglich, routinemäßig 10 fmol eines Polypeptidmarkers, also 0,1 ng eines 10 kD Proteins mit einer Messgenauigkeit von ca. ±0,01% aus einem komplexen Gemisch zu vermessen. Bei Massenspektrometern ist eine Ionen-bildende Einheit mit einem geeigneten Analysegerät gekoppelt. Zum Beispiel werden meistens Elektrosprayionisations-(ESI)-Interfaces verwendet, um Ionen aus Flüssigproben zu vermessen, wohingegen MALDI am häufigsten verwendet wird, um Ionen aus individuell hergestellten Proben herzustellen. Verschiedene Arten von Analysegeräten sind erhältlich, z.B. Ionenfallenanalysegeräte oder TOF-Analysegeräte. Sowohl ESI als auch MALDI können mit im Wesentlichen allen Typen von Massenspektrometern kombiniert werden, obwohl ESI für gewöhnlich mit Ionenfallen und MALDI mit TOF kombiniert wird.

Bei der Elektrosprayionisation (ESI) werden die in Lösung vorliegenden Moleküle u.a. unter dem Einfluss von Hochspannung (z.B. 1-8 kV) versprüht, wobei sich zunächst geladene Tröpfchen bilden, die durch Verdampfen des Lösungsmittels kleiner werden. Schließlich kommt es zur Bildung freier gasförmiger Ionen.

Bei der Massenspektrometrie mittels eines Flugzeit-Massenspektrometer (TOF) wird eine bestimmte Beschleunigungsspannung angelegt, die den Ionen eine gleich große kinetische Energie verleiht. Dann wird sehr genau die Zeit gemessen, die die jeweiligen Ionen benötigen, um eine bestimmte Driftstrecke durch das Flugrohr zurückzulegen, da bei gleicher kinetischer Energie die Geschwindigkeit der Ionen von ihrer Masse abhängt. TOF-Massenspektrometer haben eine sehr hohe Scan-Geschwindigkeit und erreichen daher eine gute Auflösung.

Bevorzugten Verfahren zur Bestimmung der An- und Abwesenheit von Polypeptidmarkern schließen Gasphasenionenspektrometrie, wie Laserdesorption/Ionisation-Massenspektrometrie, SELDI-TOF MS (surface enhanced laser desorption/ ionisation time of flight mass spectrometry), MALDI-TOF MS (matrix assisted laser desorption/ionisation time of flight mass spectrometry), 2D-PAGE/MS und Kapillarelektrophorese-Massenspektrometrie (CE-MS) ein.

2D-PAGE wird für gewöhnlich zur Auftrennung von Polypeptiden verwendet und kann mit Massenspektrometrie zur Identifikation einzelner Polypeptide verwendet werden (2D-PAGE/MS). Über 1000 Proteinpunkte können mittels 2D-PAGE unterschieden werden. Allerdings muss jeder einzelne Polypeptidpunkt getrennt durch massenspektrometrische Verfahren identifiziert werden.

In dem SELDI-System ist der Proteinchip die wichtigste Komponente. Die zu analysierenden Proben werden direkt auf die auf den Arrays befindlichen Spots aufgetragen. Es gibt verschiedene Arrays, z.B. chromatographische Arrays, die hydrophobe, hydrophile, Kationenaustauscher-, Anionenaustauscher- und immobilisierte Metallionenaffinitätsoberflächen darstellen, und voraktivierte Arrays mit chemischen Gruppen, die eine kovalente Bindung erlauben. Kationenaustauscheroberflächen sind bevorzugt. Bei dem SELDI-Verfahren werden nur die Polypeptide gemessen, die tatsächlich an die Oberfläche des Chips binden. Nach der Bindung der Proben-Polypeptide wird eine Energieabsorbierende Matrix auf jeden Spot angewandt. Nach Kristallisation der Matrix wird der Proteinchip-Array in der Regel in einem Proteinchip-Lesegerät zur Analyse ausgewertet.

Das Lesegerät ist ein TOF-(Time of Flight)-Massenspektrometer, in welchem die Proteine desorbiert und mittels Laser ionisiert werden. Da die kristallisierten Proteine auf der Spotoberfläche gleich verteilt werden, trifft der ionisierende Laserstrahl immer einen repräsentativen Durchschnitt der Moleküle in dem Analyten, was eine quantitative Bestimmung erlaubt. Nach Ionisierung werden die Polypeptide in einem elektrischen Feld beschleunigt, bevor sie auf einen Detektor treffen. Die Dauer des Flugs von der Laserbestrahlung der Array-Oberfläche bis zum Auftritt des Moleküls am Detektors erlaubt eine genaue Bestimmung der Molekularmasse des Polypeptids in der Probe (siehe z.B. den folgenden Übersichtsartikel Merchant, M. und Weinberger, S.R., Electrophoresis, Bd. 212:1164-1177,2000).

Ein besonders bevorzugtes Verfahren ist die CE-MS, in welcher die Kapillarelektrophorese mit Massenspektrometrie gekoppelt wird. Dieses Verfahren ist ausführlich z.B. in der deutschen Patentanmeldung DE 10021737, bei Kaiser et al. (J Chromatogr A, Bd. 1013:157-171, 2003 sowie Electrophoresis, 25:2044-2055, 2004) und bei Wittke et al. (Journal of Chromatography A, 1013:173-181, 2003) beschrieben. Die CE-MS-Technik erlaubt, das Vorhandensein einiger Hunderter Polypeptidmarker gleichzeitig in einer kurzen Zeit und einem geringen Volumen mit hoher Sensitivität zu bestimmen. Nachdem polypeptidhaltige Probe gemessen wurde, wird ein Muster der gemessenen Polypeptidmarker hergestellt, und dieses kann mit einem Muster eines kranken bzw. eines gesunden Menschen verglichen werden. In den meisten Fällen ist es ausreichend, nur ein oder eine begrenzte Anzahl von Polypeptidmarkern zur Diagnose von Arthritis zu verwenden. Insbesondere können 1, 2, 3, 4, 5, 6, 7, 8 oder 9 Polypeptidmarker aus Tabelle 1 oder 2, vorzugsweise in den oben angegebenen Kombinationen von Polypeptidmarkern, gemessen werden. Weiter bevorzugt ist ein CE-MS-Verfahren, das Kapillarelektrophorese, die z.B. online an ein ESI-TOF-Analysegerät gekoppelt ist, einschließt. Hier wird die Probe auf eine Elektrophoresekapillare aufgetragen und eine Spannung, z.B. bis zu 50 kV, insbesondere bis zu 30 kV, angelegt.

Für CE-MS ist die Verwendung von flüchtigen Lösungsmitteln bevorzugt, und am besten arbeitet man unter im Wesentlichen salzfreien Bedingungen. Beispiele solcher Lösungsmittel umfassen Acetonitril, Isopropanol, Methanol und ähnliche. Die Lösungsmittel können mit Wasser oder einer schwachen Säure (z.B. 0,1% Ameisensäure) gemischt werden, um den Analyten zu protonieren. Die Polypeptidemarker in der Probe werden nach ihrer Größe und Ladung aufgetrennt, die die Flugzeit in der Kapillare bestimmen.

Mithilfe der Kapillarelektrophorese ist es möglich, Moleküle nach ihrer Ladung und Größe zu trennen. Neutrale Teilchen wandern beim Anlegen eines Stroms mit der Geschwindigkeit des elektroosmotischen Flusses, Kationen werden zur Kathode beschleunigt und Anionen verzögert. Der Vorteil der Kapillaren bei der Elektrophorese besteht in dem günstigen Verhältnis von Oberfläche zu Volumen, was einen guten Abtransport der beim Stromfluss entstehenden Jouleschen Wanne ermöglicht. Dies wiederum erlaubt das Anlegen hoher Spannungen (üblicherweise bis 30 kV) und damit eine hohe Trennleistung und kurze Analysezeiten.

Bei der Kapillarelektrophorese werden in der Regel Quarzglaskapillaren verwendet. Durch Einsatz englumiger Kapillaren werden sowohl Radialdiffusion als auch Konvektion vermieden. Um bei der angelegten Spannung durch Stromfluss in der Kapillare erzeugte Wärme in ausreichendem Maß an die Umgebung abführen zu können, müssen die Innendurchmesser der Kapillaren klein gehalten werden. Es werden in der Regel Kapillaren mit Innendurchmessern von 50-75 µm eingesetzt. Die verwendeten Längen betragen z.B. 30-100 cm. Bei Kapillaren mit diesen Abmessungen ist in der Regel gewährleistet, dass die Temperaturerhöhung in der Kapillare durch freigesetzte ionische Wärme nicht zur Bildung von Gasblasen in der Kapillare führt. Darüber hinaus sind die Trennkapillaren in der Regel aus Kunststoff-umhülltem Quarzglas. Die Kapillaren können sowohl unbehandelt sein, d.h. auf der Innenseite ihre hydrophoben Gruppen zeigen, oder auch auf der Innenseite beschichtet sein. Eine hydrophobe Beschichtung kann verwendet werden, um die Auflösung zu verbessern. Zusätzlich zu der Spannung kann auch ein Druck angelegt werden, der typischerweise im Bereich von 0-1 psi liegt. Der Druck kann auch erst während der Anwendung angelegt oder währenddessen verändert werden.

Um die Auflösung zu verbessern, kann während der Beladung ein "Stacking"-Protokoll verwendet werden: Vor dem Laden der Probe wird eine Base geladen, dann die Probe, dann eine Säure. Hierdurch werden die Ionen des Analyten zwischen der Säure und der Base gehalten. Wenn eine Spannung angelegt wird, werden positiv geladene Analytionen in Richtung Base bewegt. Dort werden sie negativ geladen und in die entgegen gesetzte Richtung bewegt, wo sie positiv geladen werden. Dies wiederholt sich, bis Säuren und Basen neutralisiert sind. Man hat so eine konzentrierte Probe erhalten. Vor dem Aufladen kann die Probe mit einem geeigneten Puffer verdünnt werden.

Zur Detektion der mittels Kapillarelektrophorese getrennten Polypeptidmarker ist das Kapillarelektrophoresegerät vorzugsweise an ein Massenspektrometer gekoppelt. Die Massenspektrometrie erlaubt die Bestimmung der Molekularmassen freier Ionen im Hochvakuum. Es enthält einen Massenanalysator, der die Ionen nach ihrem Masseladungsquotienten (m/z) auftrennt, und einen Detektor.

In einem noch weiter bevorzugten Verfahren zur Messung von Polypeptidmarker in einer Probe werden die Polypeptidmarker der Probe mittels Kapillarelektrophorese getrennt, anschließend direkt ionisiert und online über ein Interface in ein daran gekoppeltes Massenspektrometer zur Detektion überführt werden.

Ein weiterer Gegenstand der vorliegenden Erfindung ist ein Verfahren zur Diagnose von Arteriosklerose
a) Messen der An- oder Abwesenheit mindestens eines Polypeptidmarkers in einer Probe eines Individuums, wobei der Polypeptidmarker wie in Anspruch 1 oder 2 definiert ist, und
b) Ermitteln der Wahrscheinlichkeit für das Vorliegen von Arteriosklerose in dem Individuum, wobei
   i) wenn die Wahrscheinlichkeit für die Anwesenheit des Polypeptidmarkers in einem kranken Individuum höher ist als die Wahrscheinlichkeit für die Anwesenheit dieses Polypeptidmarkers in einem Kontrollindividuum, dann ist die Anwesenheit des Polypeptidmarkers indikativ für eine höhere Wahrscheinlichkeit des Vorliegens von Arteriosklerose,
   ii) wenn die Wahrscheinlichkeit für die Anwesenheit des Polypeptidmarkers in einem kranken Individuum niedriger ist als die Wahrscheinlichkeit für die Anwesenheit des Polypeptidmarkers in einem Kontrollindividuum, dann ist die Abwesenheit des Polypeptidmarkers indikativ für eine höhere Wahrscheinlichkeit des Vorliegens von Arteriosklerose,
   iii) wenn die Wahrscheinlichkeit für die Anwesenheit des Polypeptidmarkers in einem kranken Individuum höher ist als die Wahrscheinlichkeit für die Anwesenheit dieses Polypeptidmarkers in einem Kontrollindividuum, dann ist die Abwesenheit des Polypeptidmarkers indikativ für eine höhere Wahrscheinlichkeit des Nichtvorliegens von Arteriosklerose, oder
   iv) wenn die Wahrscheinlichkeit für die Anwesenheit des Polypeptidmarkers in einem kranken Individuum niedriger ist als die Wahrscheinlichkeit für die Anwesenheit des Polypeptidmarkers in einem Kontrollindividuum, dann ist die Anwesenheit des Polypeptidmarkers indikativ für eine höhere Wahrscheinlichkeit des Nichtvorliegens von Arteriosklerose.

In einer Ausführungsform des erfindungsgemäßen Verfahrens sind die Wahrscheinlichkeiten in Schritt b) für die Anwesenheit eines Polypeptidmarkers in einem an Arteriosklerose erkrankten Individuum (Kranken) bzw. in einem Kontrollindividuum wie folgt sind:

| Polypeptidmarker Nr. | Molekularmasse [Da] | Migrationszeit [min] | Wahrscheinlichkeit für die Anwesenheit des Polypeptidmarkers in | |
|---|---|---|---|---|
| | | | krankem | Kontroll |
| | | | Individuum | individuum |
| 1 | 2.511,8 | 26,4 | 0,60 | 0,00 |
| 2 | 1864,7 | 49,2 | 0,20 | 0,78 |
| 3 | 2799,8 | 46,0 | 0,20 | 0,78 |
| 4 | 1340,6 | 66,7 | 0,10 | 0,67 |
| 5 | 1422,8 | 61,2 | 0,00 | 0,56 |
| 6 | 5882,0 | 33,3 | 0,00 | 0,56 |
| 7 | 1397,4 | 24,9 | 0,50 | 0,00 |
| 8 | 1886,6 | 44,7 | 0,50 | 0,00 |
| 9 | 4642,6 | 37,5 | 0,50 | 0,00 |

In einer bevorzugten Ausführungsform stellt das Kontrollindividuum ein nicht an Arteriosklerose erkranktes Individuum dar. In einer noch weiter bevorzugten Ausführungsform handelt es sich um einen nicht an Arteriosklerose erkrankten Menschen.

In dem erfindungsgemäßen Verfahren können vorteilhafter Weise mehrere Polypeptidmarker zur Diagnose verwendet werden. Insbesondere können mindestens 2, 3, 4, 5, 6, 7, 8 oder 9 Polypeptidmarker der Tabelle 1 oder Tabelle 2 verwendet werden. Noch weiter bevorzugt handelt es sich bei den Kombinationen von Polypeptidmarkern um die oben aufgeführten Kombinationen.

Um die Wahrscheinlichkeit für das Vorliegen von Arteriosklerose bei Verwendung mehrerer Marker zu bestimmen, können dem Fachmann bekannte statistische Verfahren verwendet werden. Beispielsweise kann das von Weissinger et al. (Kidney Int. 2004 Jim; 65(6): 2426-24) beschriebene Verfahren (Random Forests-Verfahren) unter Verwendung eines Computerprogramms wie z.B. S-Plus verwendet werden.

In dem erfindungsgemäßen Verfahren ist die Anwesenheit der Polypeptidmarker Nr. 1, Nr. 7, Nr. 8 und/oder Nr. 9 in der Probe und/oder die Abwesenheit der Polypeptidmarker Nr. 2, Nr. 3, Nr. 4, Nr. 5 und/oder Nr. 6 in der Probe indikativ für das Vorliegen von Arteriosklerose.

Zur Bestimmung der An- oder Abwesenheit kann in dem erfindungsgemäßen Verfahren können die oben genannten Reinigungs-, Trennungs- und Nachweisverfaliren eingesetzt werden. Vorzugsweise wird Kapillarelektrophorese, Gasphasenionenspektrometrie und/oder Massenspektrometrie zum Nachweis der An- oder Abwesenheit des oder der Polypeptidmarker(s) verwendet wird.

In einer besonders bevorzugten Ausführungsform wird vor der Messung der Molekularmasse der Polypeptidmarker eine Kapillarelektrophorese durchgeführt und/oder die Massenspektrometrie zum Nachweis der An- oder Abwesenheit des/der Polypeptidmarker(s) verwendet wird.

### Beispiel:

### 1. Probenvorbereitung

Zur Detektion der Polypeptidmarker für Arteriosklerose wurde Plasma verwendet. Plasma wurde von gesunden Spendern (Kontrolle) sowie von dialysepflichtigen Patienten, die an Arteriosklerose leiden, abgenommen. Zur Abnahme des Plasmas von gesunden Spendern wurden S-Monovetten mit Heparin (Sarstedt, Nürnbrecht, DE) verwendet, Plasma von Dialysepatienten wurde direkt abgenommen, da diesen bereits Heparin verabreicht wurde. Heparin dient hierbei zur Vermeidung der Blutgerinnung und von proteolytischer Aktivität und damit auch zum Schutz der im Plasma enthaltenen Proteine und Peptide vor Abbau durch Enzyme.

Für die nachfolgende CE-MS Messung mussten die im Plasma in sehr hoher Konzentration vorkommenden Proteine wie Albumin und Immunoglobuline durch Ultrafiltration abgetrennt werden. Dazu wurden 500 µl Plasma entnommen und mit 2 ml Filtrationspuffer (4 mol/l Harnstoff, 0,1 mol/l NaCl, 0,01% Ammoniak) versetzt. Diese 2,5 ml Probenvolumen wurden ultrafiltriert (Centrisart 20 MWCO, Vivascience, Hannover, DE). Die Ultrafiltration wurde bei 3400 U/min in einer Zentrifuge durchgeführt bis 2 ml Ultrafiltrat erhalten wurden.

Die erhaltenen 2 ml Filtrat wurden dann auf eine Pharmacia C-2 Säule aufgetragen (Pharmacia, Uppsala, Schweden), um Harnstoff, Salze und andere störende Komponenten zu entfernen. Die gebundenen Polypeptide wurden dann mit 50% Acetonitril, 0,5% Ameisensäure in Wasser von der C-2 Säule eluiert und lyophillisiert. Zur CE-MS Messung wurden die Polypeptide dann mit 20 µl Wasser (HPLC-Reinheit, Merck, Darmstadt, DE) resuspendiert.

### 2. CE-MS Messung

Die CE-MS Messungen wurden mit einem Kapillarelektrophoresesystem von Beckman Coulter (P/ACE MDQ System; Beckman Coulter Inc., Fullerton, CA, USA) und einem ESI-TOF Massenspektrometer von Applied Biosysteins (Mariner Biospectrometry Workstation; Applied Biosystems, Foster City, CA, USA) durchgeführt.

Die CE Kapillaren wurden von Beckman Coulter (supra) bezogen, sie hatten einen ID/OD von 75/360 µm und eine Länge von 90 cm. Die mobile Phase für die CE Trennung bestand aus 30% Methanol und 0,5% Ameisensäure in Wasser, die gleiche Mischung wurde für den "Sheath-Flow" am MS verwendet, hier mit einer Flussrate von 2 µl/min. Die Kopplung von CE und MS wurde durch ein CE-ESI-MS Sprayer Kit (Agilent Technologies, Waldbronn, DE) realisiert. Um die Probe zu injizieren, wurde 1 psi Druck angelegt, die Dauer der Injektion betrug 20 Sekunden. Mit diesen Parametern wurden ca. 100 nl der Probe in die Kapillare injiziert, dieses entspricht ca. 0,25% des Kapillarvolumens. Um die Probe in der Kapillare aufzukonzentrieren wurde eine "Stacking"-Technik verwendet. Dabei wurde vor der Probeninjektion für 7 Sek. (bei 1 psi) eine 1 mol/l NH3 Lösung injiziert, nach der Probeninjektion für 5 Sek. eine 2 mol/l Ameisensäurelösung. Bei Anlegen der Trennspannung (30 kV) wurden die Analyten zwischen diesen Lösungen automatisch aufkonzentriert.

Die folgende CE-Trennung wurde mit einer Druckmethode durchgeführt: 40 Minuten mit 0 psi, dann für 2 min 0,1 psi, für 2 min 0,2 psi, für 2 min 0,3 psi, für 2 min 0,4 psi, abschließend 32 min bei 0,5 psi. Die Gesamtdauer eines Trennlaufes betrug damit 80 Minuten.

Um auf der Seite des MS eine möglichst gute Signalintensität zu erhalten, wurde das Nebulizer Gas abgestellt. Die an der Spraynadel angelegte Spannung zur Erzeugung des Elektrosprays betrug 3200 V, woraus eine verfügbare Trennspannung über die gesamte Kapillare von ca. 27 kV resultierte. Die übrigen Einstellungen am Mariner Massenspektrometer wurden gemäß Anweisung des Herstellers für Peptiddetektion optimiert. Die Spektren wurden über einen Massenbereich von m/z 400 bis m/z 2500 aufgenommen und alle 3 Sek. akkumuliert.

### 3. Standards für die CE-Messung

Zur Kontrolle und Standardisierung der CE-Messung wurden die folgenden Proteine bzw. Polypeptide eingesetzt, welche durch die aufgeführten CE-Migrationszeiten charakterisiert sind:
Diese können zur Normierung der der CE-Zeiten der Peptidmarker 1 bis 9 sowie gemessener Proben verwendet werden.
Protein/Polypeptid Migrationszeit
Aprotinin (SIGMA, Taufkirchen, DE, Kat-Nr. AI 153) 9,2 min
Ribonuclease (SIGMA, Taufkirchen, DE, Kat-Nr. R4875) 10,9 min
Lysozym (SIGMA, Taufkirchen, DE, Kat-Nr. L7651) 8,9 min
"REV", Sequenz: REVQSKIGYGRQIIS 15,6 min
"ELM", Sequenz: ELMTGELPYSHINNRDQIIFMVGR 23,4 min
"KINCON", Sequenz: TGSLPYSHIGSRDQIIFMVGR 20,0 min
"GIVLY" Sequenz: GIVLYELMTGELPYSHIN 36,8 min
Die Proteine/Polypeptide werden jeweils in einer Konzentration von 10 pmol/µl in Wasser eingesetzt.
"REV", "ELM, "KINCON" und "GIVLY" stellen synthetische Peptide dar.
Die Molekularmassen der Peptide sowie die in der MS sichtbaren Massen der einzelnen Ladungszustände sind wie folgt:

| **H mono** | 1,0079 | 1,0079 | 1,0079 | 1,0079 | 1,0079 | 1,0079 | 1,0079 |
|---|---|---|---|---|---|---|---|
| m/z | Aprotinin | Ribonuclease | Lysozym | REV | KINCON | ELM | GIVLY |
| | Mono Mass | Mono Mass | Mono Mass | Mono Mass | Mono Mass | Mono Mass | Mono Mass |
| 0 | 6513,0900 | 13681,3200 | 14303,8800 | 1732,9600 | 2333,1900 | 2832,4100 | 2048,0300 |
| 1 | 6514,0979 | 13682,3279 | 14304,8879 | 1733,9679 | 2334,1979 | 2833,4179 | 2049,0379 |
| 2 | 3257,5529 | 6841,6679 | 7152,9479 | 867,4879 | 1167,6029 | 1417,2129 | 1025,0229 |
| 3 | 2172,0379 | 4561,4479 | 4768,9679 | 578,6612 | 778,7379 | 945,1446 | 683,6846 |
| 4 | 1629,2804 | 3421,3379 | 3576,9779 | 434,2479 | 584,3054 | 709,1104 | 513,0154 |
| 5 | 1303,6259 | 2737,2719 | 2861,7839 | 347,5999 | 467,6459 | 567,4899 | 410,6139 |
| 6 | 1086,5229 | 2281,2279 | 2384,9879 | 289,8346 | 389,8729 | 473,0762 | 342,3462 |
| 7 | 931,4494 | 1955,4822 | 2044,4193 | 248,5736 | 334,3208 | 405,6379 | 293,5836 |
| 8 | 815,1442 | 1711,1729 | 1788,9929 | 217,6279 | 292,6567 | 355,0592 | 257,0117 |
| 9 | 724,6846 | 1521,1546 | 1590,3279 | 193,5590 | 260,2512 | 315,7201 | 228,5668 |
| 10 | 652,3169 | 1369,1399 | 1431,3959 | 174,3039 | 234,3269 | 284,2489 | 205,8109 |
| 11 | 593,1070 | 1244,7643 | 1301,3606 | 158,5497 | 213,1161 | 258,4997 | 187,1924 |
| 12 | 543,7654 | 1141,1179 | 1192,9979 | 145,4212 | 195,4404 | 237,0421 | 171,6771 |
| 13 | 502,0148 | 1053,4171 | 1101,3063 | 134,3125 | 180,4841 | 218,8856 | 158,5486 |

## Patentansprüche

1. Verwendung der An- oder Abwesenheit mindestens eines Polypeptidmarkers in der Probe eines Individuums, wobei der Polypeptidmarker ausgewählt ist aus den Polpeptidmarkern Nr. 1, Nr. 2, Nr. 3, Nr. 4, Nr. 5, Nr. 6, Nr. 7, Nr. 8 oder Nr. 9, die durch die folgenden Werte für die Molekülmasse und die Migrationszeit charakterisiert sind:
| Polypeptidmarker Nr. | Molekülmasse [Da] | Migrationszeit [min] |
|---|---|---|
| 1 | 2511,8 | 26,4 |
| 2 | 1864,7 | 49,2 |
| 3 | 2799,8 | 46,0 |
| 4 | 1340,6 | 66,7 |
| 5 | 1422,8 | 61,2 |
| 6 | 5882,0 | 33,3 |
| 7 | 1397,4 | 24,9 |
| 8 | 1886,6 | 44,7 |
| 9 | 4642,6 | 37,5 |
zur Diagnose von Arteriosklerose, wobei die Migrationszeit durch Kapillarelektrophorese bei 30 kV in einer mobilen Phase von 30 Vol% Methanol, 0.5 Vol% Ameisensäure in Wasser in einer Kapillare mit einer Länge von 90 cm bestimmt wurde.

2. Verwendung nach Anspruch 1, wobei mindestens zwei Polypeptidmarker verwendet werden, insbesondere die Polypeptidmarker:
- Nr. 1 und Nr. 2, Nr. 1 und Nr. 3, Nr. 1 und Nr. 4, Nr. 1 und Nr. 5, Nr. 1 und Nr.6, Nr. 1 und Nr. 7, Nr. 1 und Nr. 8, Nr. 1 und Nr. 9,
- Nr. 2 und Nr. 3, Nr. 2 und Nr. 4, Nr. 2 und Nr. 5, Nr. 2 und Nr. 6, Nr. 2 und Nr.7, Nr. 2 und Nr. 8, Nr. 2 und Nr. 9,
- Nr. 3 und Nr. 4, Nr. 3 und Nr. 5, Nr. 3 und Nr. 6, Nr. 3 und Nr. 7, Nr. 3 und Nr. 8, Nr. 3 und Nr. 9,
- Nr. 4 und Nr. 5, Nr. 4 und Nr. 6, Nr. 4 und Nr. 7, Nr. 4 und Nr. 8, Nr. 4 und Nr. 9,
- Nr. 5 und Nr. 6, Nr. 5 und Nr. 7, Nr. 5 und Nr. 8, Nr. 5 und Nr. 9,
- Nr. 6 und Nr. 7, Nr. 6 und Nr. 8, Nr. 6 und Nr. 9,
- Nr. 7 und Nr. 8, Nr. 7 und Nr. 9 oder
- Nr. 8 und Nr. 9,
und wobei die Polypeptidmarker wie in Anspruch 1 definiert sind.

3. Verwendung nach Anspruch 1 oder 2, wobei mindestens drei, vier, fünf, sechs, sieben, acht oder neun Polypeptidmarker verwendet werden, die wie in Anspruch 1 oder 2 definiert sind.

4. Verwendung nach einem der Ansprüche 1 bis 3, wobei mindestens ein weiteres Polypeptid als Marker verwendet wird.

5. Verwendung nach einem der Ansprüche 1 bis 4, wobei die Probe eines Individuums eine Urinprobe oder eine Blutprobe, insbesondere eine Serum- oder Plasmaprobe, ist.

6. Verfahren zur Diagnose von Arteriosklerose umfassend die Schritte:
a) Messen der An- oder Abwesenheit mindestens eines Polypeptidmarkers in einer Probe eines Individuums, wobei der Polypeptidmarker wie in Anspruch 1 definiert ist, und
b) Ermitteln der Wahrscheinlichkeit für das Vorliegen von Arteriosklerose in dem Individuum, wobei
i) wenn die Wahrscheinlichkeit für die Anwesenheit des Polypeptidmarkers in einem kranken Individuum höher ist als die Wahrscheinlichkeit für die Anwesenheit dieses Polypeptidmarkers in einem Kontrollindividuum, dann ist die Anwesenheit des Polypeptidmarkers indikativ für eine höhere Wahrscheinlichkeit des Vorliegens von Arteriosklerose,
ii) wenn die Wahrscheinlichkeit für die Anwesenheit des Polypeptidmarkers in einem kranken Individuum niedriger ist als die Wahrscheinlichkeit für die Anwesenheit des Polypeptidmarkers in einem Kontrollindividuum, dann ist die Abwesenheit des Polypeptidmarkers indikativ für eine höhere Wahrscheinlichkeit des Vorliegens von Arteriosklerose,
iii) wenn die Wahrscheinlichkeit für die Anwesenheit des Polypeptidmarkers in einem kranken Individuum höher ist als die Wahrscheinlichkeit für die Anwesenheit dieses Polypeptidmarkers in einem Kontrollindividuum, dann ist die Abwesenheit des Polypeptidmarkers indikativ für eine höhere Wahrscheinlichkeit des Nichtvorliegens von Arteriosklerose, oder
iv) wenn die Wahrscheinlichkeit für die Anwesenheit des Polypeptidmarkers in einem kranken Individuum niedriger ist als die Wahrscheinlichkeit für die Anwesenheit des Polypeptidmarkers in einem Kontrollindividuum, dann ist die Anwesenheit des Polypeptidmarkers indikativ für eine höhere Wahrscheinlichkeit des Nichtvorliegens von Arteriosklerose.

7. Verfahren nach Anspruch 6, wobei die einzelnen Wahrscheinlichkeiten in Schritt b) wie folgt sind:
| Polypeptidmarker Nr. | Wahrscheinlichkeit für die Anwesenheit des Polypeptidmarkers in | |
|---|---|---|
| | krankem Individuum | Kontrollindividuum |
| 1 | 0,60 | 0,00 |
| 2 | 0,20 | 0,78 |
| 3 | 0,20 | 0,78 |
| 4 | 0,10 | 0,67 |
| 5 | 0,00 | 0,56 |
| 6 | 0,00 | 0,56 |
| 7 | 0,50 | 0,00 |
| 8 | 0,50 | 0,00 |
| 9 | 0,50 | 0,00 |

8. Verfahren nach Anspruch 6 oder 7, wobei mindestens 2, 3, 4, 5, 6, 7, 8 oder 9 Polypeptidmarker verwendet werden.

9. Verfahren nach einem der Ansprüche 6 bis 8, wobei eine Kombination von Polypeptidmarkern, wie in Anspruch 3 definiert, verwendet wird.

10. Verfahren nach einem der Ansprüche 6 bis 9, wobei die Anwesenheit der Polypeptidmarker Nr. 1, Nr. 7, Nr. 8 und/oder Nr. 9 indikativ für das Vorliegen von Arteriosklerose ist.

11. Verfahren nach einem der Ansprüche 6 bis 10, wobei die Abwesenheit der Polypeptidmarker Nr. 2, Nr. 3, Nr. 4, Nr. 5 und/oder Nr. 6 indikativ für das Vorliegen von Arteriosklerose ist.

12. Verfahren nach einem der Ansprüche 6 bis 11, wobei Kapillarelektrophorese, Gasphasenionenspektrometrie und/oder Massenspektrometrie zum Nachweis der An- oder Abwesenheit des/der Polypeptidmarker(s) verwendet wird.

13. Verfahren nach einem der Ansprüche 6 bis 12, wobei vor der Messung der Molekularmasse der Polypeptidmarker eine Kapillarelektrophorese durchgeführt wird.

14. Verfahren nach einem der Ansprüche 6 bis 13, wobei Massenspektrometrie zum Nachweis der An- oder Abwesenheit des/der Polypeptidmarker(s) verwendet wird.

## Claims

1. Use of the presence or absence of at least one polypeptide marker in a sample from an individual, wherein said polypeptide marker is selected from polypeptide markers No. 1, No. 2, No. 3, No. 4, No. 5, No. 6, No. 7, No. 8 or No. 9, which are **characterized by** the following values of molecular mass and migration time:
| Polypeptide marker No. | Molecular mass [Da] | Migration time [min] |
|---|---|---|
| 1 | 2511.8 | 26.4 |
| 2 | 1864.7 | 49.2 |
| 3 | 2799.8 | 46.0 |
| 4 | 1340.6 | 66.7 |
| 5 | 1422.8 | 61.2 |
| 6 | 5882.0 | 33.3 |
| 7 | 1397.4 | 24.9 |
| 8 | 1886.6 | 44.7 |
| 9 | 4642.6 | 37.5 |
for the diagnosis of arteriosclerosis, wherein said migration time is determined by capillary electrophoresis at 30 kV in a mobile phase of 30% by volume methanol, 0.5% by volume formic acid in water in a capillary having a length of 90 cm.

2. The use according to claim 1, wherein at least two polypeptide markers are used, especially the polypeptide markers:
- No. 1 and No. 2, No. 1 and No. 3, No. 1 and No. 4, No. 1 and No. 5, No. 1 and No. 6, No. 1 and No. 7, No. 1 and No. 8, No. 1 and No. 9;
- No. 2 and No. 3, No. 2 and No. 4, No. 2 and No. 5, No. 2 and No. 6, No. 2 and No. 7, No. 2 and No. 8, No. 2 and No. 9;
- No. 3 and No. 4, No. 3 and No. 5, No. 3 and No. 6, No. 3 and No. 7, No. 3 and No. 8, No. 3 and No. 9;
- No. 4 and No. 5, No. 4 and No. 6, No. 4 and No. 7, No. 4 and No. 8, No. 4 and No. 9;
- No. 5 and No. 6, No. 5 and No. 7, No. 5 and No. 8, No. 5 and No. 9;
- No. 6 and No. 7, No. 6 and No. 8, No. 6 and No. 9;
- No. 7 and No. 8, No. 7 and No. 9; or
- No. 8 and No. 9;
and wherein said polypeptide markers are as defined in claim 1.

3. The use according to claim 1 or 2, wherein at least three, four, five, six, seven, eight or nine polypeptide markers as defined in claim 1 or 2 are used.

4. The use according to any of claims 1 to 3, wherein at least one further polypeptide is used as a marker.

5. The use according to any of claims 1 to 4, wherein said sample from an individual is a urine sample or blood sample, especially a serum or plasma sample.

6. A process for the diagnosis of arteriosclerosis, comprising the steps of:
a) measuring the presence or absence of at least one polypeptide marker in a sample from an individual, wherein said polypeptide marker is as defined in claim 1; and
b) establishing the probability of the existence of arteriosclerosis in the individual, wherein
i) if the probability of the presence of said polypeptide marker in a sick individual is higher than the probability of the presence of said polypeptide marker in a control individual, then the presence of said polypeptide marker is indicative of a higher probability of the existence of arteriosclerosis;
ii) if the probability of the presence of said polypeptide marker in a sick individual is lower than the probability of the presence of said polypeptide marker in a control individual, then the absence of said polypeptide marker is indicative of a higher probability of the existence of arteriosclerosis;
iii) if the probability of the presence of said polypeptide marker in a sick individual is higher than the probability of the presence of said polypeptide marker in a control individual, then the absence of said polypeptide marker is indicative of a higher probability of the non-existence of arteriosclerosis; or
iv) if the probability of the presence of said polypeptide marker in a sick individual is lower than the probability of the presence of said polypeptide marker in a control individual, then the presence of said polypeptide marker is indicative of a higher probability of the non-existence of arteriosclerosis.

7. The process according to claim 6, wherein the individual probabilities in step b) are as follows:
| Polypeptide marker No. | Probability of the presence of the polypeptide marker in | |
|---|---|---|
| | sick individual | control individual |
| 1 | 0.60 | 0.00 |
| 2 | 0.20 | 0.78 |
| 3 | 0.20 | 0.78 |
| 4 | 0.10 | 0.67 |
| 5 | 0.00 | 0.56 |
| 6 | 0.00 | 0.56 |
| 7 | 0.50 | 0.00 |
| 8 | 0.50 | 0.00 |
| 9 | 0.50 | 0.00 |

8. The process according to claim 6 or 7, wherein at least 2, 3, 4, 5, 6, 7, 8 or 9 polypeptide markers are used.

9. The process according to any of claims 6 to 8, wherein a combination of polypeptide markers as defined in claim 3 is used.

10. The process according to any of claims 6 to 9, wherein the presence of polypeptide markers No. 1, No. 7, No. 8 and/or No. 9 is indicative of the existence of arteriosclerosis.

11. The process according to any of claims 6 to 10, wherein the absence of polypeptide markers No. 2, No. 3, No. 4, No. 5 and/or No. 6 is indicative of the existence of arteriosclerosis.

12. The process according to any of claims 6 to 11, wherein capillary electrophoresis, gas-phase ion spectrometry and/or mass spectrometry is used for detecting the presence or absence of the polypeptide marker or markers.

13. The process according to any of claims 6 to 12, wherein a capillary electrophoresis is performed before the molecular masses of the polypeptide markers are measured.

14. The process according to any of claims 6 to 13, wherein mass spectrometry is used for detecting the presence or absence of the polypeptide marker or markers.

## Revendications

1. Utilisation de la présence ou de l'absence d'au moins un marqueur polypeptidique dans l'échantillon d'un individu, le marqueur polypeptidique étant sélectionné parmi les marqueurs polypeptidiques n° 1, n° 2, n° 3, n° 4, n° 5, n° 6, n° 7, n° 8 ou n° 9 qui sont **caractérisés par** les valeurs suivantes pour la masse moléculaire et le temps de migration :
| Marqueur | Masse moléculaire | Temps de |
|---|---|---|
| polypeptidique n° | [Da] | migration [min] |
| 1 | 2 511,8 | 26,4 |
| 2 | 1 864,7 | 49,2 |
| 3 | 2 799,8 | 46,0 |
| 4 | 1 340,6 | 66,7 |
| 5 | 1 422,8 | 61,2 |
| 6 | 5 882,0 | 33,3 |
| 7 | 1 397,4 | 24,9 |
| 8 | 1 886,6 | 44,7 |
| 9 | 4 642,6 | 37,5 |
pour le diagnostic de l'artériosclérose, le temps de migration ayant été déterminé par électrophorèse capillaire à 30 kV dans une phase mobile de 30 % en vol. de méthanol, 0,5 % en vol. d'acide formique dans de l'eau dans un capillaire d'une longueur de 90 cm.

2. Utilisation selon la revendication 1, au moins deux marqueurs polypeptidiques étant utilisés, en particulier les marqueurs polypeptidiques :
- n° 1 et n °2, n° 1 et n °3, n° 1 et n° 4, n° 1 et n°5, n° 1 et n° 6, n° 1 et n° 7, n° 1 et n° 8, n° 1 et n° 9,
- n° 2 et n° 3, n° 2 et n° 4, n° 2 et n° 5, n° 2 et n° 6, n° 2 et n°7, n° 2 et n° 8, n° 2 et n° 9,
- n° 3 et n° 4, n° 3 et n° 5, n° 3 et n° 6, n° 3 et n° 7, n° 3 et n° 8, n° 3 et n° 9,
- n° 4 et n° 5, n° 4 et n° 6, n° 4 et n° 7, n° 4 et n° 8, n° 4 et n° 9,
- n° 5 et n° 6, n° 5 et n° 7, n° 5 et n° 8, n° 5 et n°9,
- n° 6 et n° 7, n° 6 et n° 8, n° 6 et n° 9,
- n° 7 et n° 8, n° 7 et n° 9,
- n° 8 et n° 9,
les marqueurs polypeptidiques étant comme définis dans la revendication 1.

3. Utilisation selon la revendication 1 ou 2, au moins trois, quatre, cinq, six, sept, huit ou neuf marqueurs polypeptidiques étant utilisés, qui sont définis comme dans la revendication 1 ou 2.

4. Utilisation selon l'une des revendications 1 à 3, au moins un autre polypeptide étant utilisé comme marqueur.

5. Utilisation selon l'une des revendications 1 à 4, l'échantillon d'un individu étant un échantillon d'urine ou un échantillon de sang, en particulier un échantillon de sérum ou de plasma.

6. Procédé de diagnostic de l'artériosclérose, comprenant les étapes suivantes :
a) mesure de la présence ou de l'absence d'au moins un marqueur polypeptidique dans un échantillon d'un individu, le marqueur polypeptidique étant comme défini dans la revendication 1, et
b) détermination de la probabilité de la présence d'artériosclérose chez l'individu, avec les scénarios suivants
i) si la probabilité de la présence du marqueur polypeptidique chez un individu malade est supérieure à la probabilité de la présence de ce marqueur polypeptidique chez un individu témoin, alors la présence du marqueur polypeptidique indique une probabilité plus élevée de l'existence de l'artériosclérose
ii) si la probabilité de la présence du marqueur polypeptidique chez un individu malade est inférieure à la probabilité de la présence du marqueur polypeptidique chez un individu témoin, alors l'absence du marqueur polypeptidique indique une probabilité plus élevée de l'existence de l'artériosclérose,
iii) si la probabilité de la présence du marqueur polypeptidique chez un individu malade est supérieure à la probabilité de la présence de ce marqueur polypeptidique chez un individu témoin, alors l'absence du marqueur polypeptidique indique une probabilité plus élevée de la non-existence d'artériosclérose, ou
iv) si la probabilité de la présence du marqueur polypeptidique chez un individu malade est inférieure à la probabilité de la présence du marqueur polypeptidique chez un individu témoin, alors la présence du marqueur polypeptidique indique une probabilité plus élevée de la non-existence d'artériosclérose.

7. Procédé selon la revendication 6, les différentes probabilités dans l'étape b) étant comme suit :
| Marqueur polypeptidique n° | Probabilité de présence du marqueur polypeptidique chez | |
|---|---|---|
| | un individu malade | un individu témoin |
| 1 | 0,60 | 0,00 |
| 2 | 0,20 | 0,78 |
| 3 | 0,20 | 0,78 |
| 4 | 0,10 | 0,67 |
| 5 | 0,00 | 0,56 |
| 6 | 0,00 | 0,56 |
| 7 | 0,50 | 0,00 |
| 8 | 0,50 | 0,00 |
| 9 | 0,50 | 0,00 |

8. Procédé selon la revendication 6 ou 7, au moins 2, 3, 4, 5, 6, 7, 8 ou 9 marqueurs polypeptidiques étant utilisés.

9. Procédé selon l'une des revendications 6 à 8, une combinaison de marqueurs polypeptidiques, comme définis dans la revendication 3, étant utilisée.

10. Procédé selon l'une des revendications 6 à 9, la présence des marqueurs polypeptidiques n° 1, n° 7, n° 8 et/ou n° 9 indiquant l'existence d'artériosclérose.

11. Procédé selon l'une des revendications 6 à 10, l'absence des marqueurs polypeptidiques n° 2, n° 3, n° 4, n° 5 et/ou n° 6 indiquant l'existence d'artériosclérose.

12. Procédé selon l'une des revendications 6 à 11, l'électrophorèse capillaire, la spectrométrie ionique en phase gazeuse et/ou la spectrométrie de masse étant utilisées pour démontrer la présence ou l'absence du/des marqueur(s) polypeptidique(s).

13. Procédé selon l'une des revendications 6 à 12, une électrophorèse capillaire étant effectuée avant la mesure de la masse moléculaire des marqueurs polypeptidiques.

14. Procédé selon l'une des revendications 6 à 13, la spectrométrie de masse étant utilisée pour démontrer la présence ou l'absence du/des marqueur(s) polypeptidique(s).
